(19) Europäisches Patentamt
European Patent Office
Office européen des brevets

(11) **EP 0 859 233 B1**

(12) **EUROPEAN PATENT SPECIFICATION**

(45) Date of publication and mention
of the grant of the patent:
**29.10.2008   Bulletin 2008/44**

(51) Int Cl.:
***G01N 27/407*** *(2006.01)*    ***G01N 27/417*** *(2006.01)*

(21) Application number: **98300999.4**

(22) Date of filing: **11.02.1998**

(54) **Gas sensor**

Gassensor

Capteur de gaz

(84) Designated Contracting States:
**DE FR GB IT SE**

(30) Priority: **12.02.1997   JP 2817497**

(43) Date of publication of application:
**19.08.1998   Bulletin 1998/34**

(73) Proprietor: **NGK INSULATORS, LTD.
Nagoya City, Aichi Pref. (JP)**

(72) Inventors:
  • **Kato, Nobuhide
   Ama-gun,
   Aichi-pref. 497 (JP)**

  • **Nakagaki, Kunihiko
   Nagoya-city,
   Aichi-pref. 461 (JP)**

(74) Representative: **Paget, Hugh Charles Edward et al
   Mewburn Ellis LLP
   York House
   23 Kingsway
   London WC2B 6HP (GB)**

(56) References cited:
**EP-A- 0 257 842        EP-A- 0 678 740
EP-A- 0 731 351        EP-A- 0 797 095
EP-A- 0 798 555        EP-A- 0 798 556**

EP 0 859 233 B1

**Description**

BACKGROUND OF THE INVENTION

Field of the Invention:

The present invention relates to a gas sensor for measuring gas components such as NO, $NO_2$, $SO_2$, $CO_2$, and $H_2O$ contained, for example, in atmospheric air and exhaust gas discharged from vehicles or automobiles.

Description of the Related Art:

Exhaust gas, which is discharged from vehicles or automobiles such as gasoline-fueled automobiles and diesel powered automobiles, contains nitrogen oxides (NOx) such as nitrogen monoxide (NO) and nitrogen dioxide ($NO_2$), as well as carbon monoxide (CO), carbon dioxide ($CO_2$), water ($H_2O$), hydrocarbon (HC), hydrogen ($H_2$), oxygen ($O_2$) and so on. In such exhaust gas, about 80 % of the entire NOx is occupied by NO, and about 95 % of the entire NOx is occupied by NO and $NO_2$.

The three way catalyst, which is used to clean HC, CO, and NOx contained in the exhaust gas, exhibits its maximum cleaning efficiency in the vicinity of the theoretical air fuel ratio (A/F = 14.6). If A/F is controlled to be not less than 16, the amount of produced NOx is decreased. However, the cleaning efficiency of the catalyst is lowered, and consequently the amount of discharged NOx is apt to increase.

Recently, in order to effectively utilize fossil fuel and avoid global warming, the market demand increases, for example, in that the discharge amount of $CO_2$ should be suppressed. In order to respond to such a demand, it becomes more necessary to improve the fuel efficiency. In response to such a demand, for example, the lean burn engine and the catalyst for cleaning NOx are being researched. Especially, the need for a NOx sensor increases.

A conventional NOx analyzer has been hitherto known in order to be used to detect NOx as described above. The conventional NOx analyzer is operated to measure a characteristic inherent in NOx, based on the use of chemical luminous analysis. However, the conventional NOx analyzer is inconvenient in that the instrument itself is extremely large and expensive. The conventional NOx analyzer requires frequent maintenance because optical parts are used to detect NOx. Further, when the conventional NOx analyzer is used, any sampling operation should be performed for measurement of NOx, wherein it is impossible to directly insert a detecting element itself into a fluid. Therefore, the conventional NOx analyzer is not suitable for analyzing transient phenomena such as those occur in the exhaust gas discharged from an automobile, in which the condition frequently varies.

In order to dissolve the inconveniences as described above, there has been suggested a sensor for measuring a desired gas component in exhaust gas by using a substrate composed of an oxygen ion-conductive solid electrolyte.

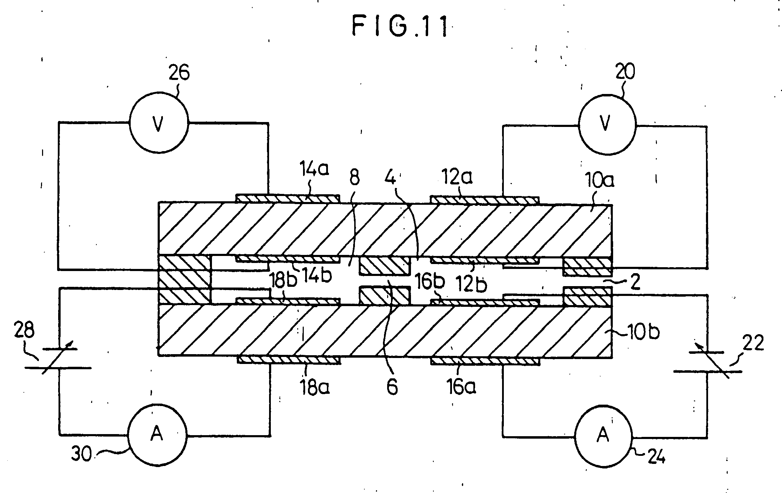

FIG. 11 shows a cross-sectional arrangement of a gas analyzer disclosed in International Publication WO 95/30146. This apparatus comprises a first chamber 4 for introducing a measurement gas containing NO through a small hole 2 thereinto, and a second chamber 8 for introducing the measurement gas from the first chamber 4 through a small hole 6. Wall surfaces for constructing the first chamber 4 and the second chamber 8 are composed of zirconia ($ZrO_2$) partition walls 10a, 10b through which oxygen ion is transmittable. A pair of measuring electrodes 12a, 12b, 14a, 14b for detecting the partial pressure of oxygen in the respective chambers are disposed on one of the $ZrO_2$ partition walls 10a of the first chamber 4 and the second chamber 8 respectively. Pumping electrodes 16a, 16b, 18a, 18b for pumping out $O_2$ in the respective chambers to the outside of the chambers are disposed on the other $ZrO_2$ partition wall 10b respectively.

In the gas analyzer constructed as described above, the partial pressure of oxygen contained in the measurement gas introduced into the first chamber 4 via the small hole 2 is detected by a voltmeter 20 as a difference in electric potential generated between the measuring electrodes 12a, 12b. A voltage in a range of 100 to 200 mV is applied between the pumping electrodes 16a, 16b by the aid of a power source 22 so that the difference in electric potential has a predetermined value. Accordingly, $O_2$ in the first chamber 4 is pumped out to the outside of the apparatus. The amount of oxygen pumped out as described above can be measured by using an ammeter 24.

On the other hand, the measurement gas, from which almost all of $O_2$ has been removed, is introduced into the second chamber 8 via the small hole 6. In the second chamber 8, a difference in electric potential, which is generated between the measuring electrodes 14a, 14b, is detected by using a voltmeter 26. Thus, the partial pressure of oxygen in the second chamber 8 is measured. Further, NO contained in the measurement gas introduced into the second chamber 8 is decomposed as follows by the aid of the voltage applied between the pumping electrodes 18a, 18b by means of a power source 28:

$$NO \rightarrow (1/2)N_2 + (1/2)O_2$$

$O_2$ is generated during this process, which is pumped out to the outside of the chamber by the aid of the pumping electrodes 18a, 18b. At this time, a generated current value is detected by using an ammeter 30. Thus, the concentration of NO contained in the measurement gas is measured.

It is considered to be desirable that an ally having a low NOx-decomposing ability comprising Au or comprising Au of a content of 1 % and the balance of Pt is used at least for the inner pumping electrode 16b of the oxygen pump based on the use of the electrochemical cell provided for the first chamber 4.

However, such an electrode containing only Au or containing an alloy composed of Au is inferior in heat resistance. It is feared that if such an electrode is used for a long period of time, the electrode function is lowered due to sintering, resulting in insufficient operation as an oxygen pump.

EP-A-678740 describes gas sensors which have the features of the first part of claim 1 and the first part of claim 3.

EP-A-0731351 discloses a cermet electrode composed of an alloy of Au and a metal of the platinum group and ceramic component, Au being contained in said alloy in a ratio of not less than 0.3 wt% and not more than 35 wt%.

SUMMARY OF THE INVENTION

The present invention has been made in order to dissolve the drawback involved in the conventional measurement of nitrogen oxide, a task to be solved or an object of which is to provide a gas sensor which makes it possible to suppress the reaction of $NO + (1/2)O_2 \rightarrow NO_2$ on the electrode of the main pumping means, and measure, for example, the NOx concentration in a measurement gas in a stable manner for a long period of time without being affected, for example, by oxygen, $CO_2$, and $H_2O$.

The present invention in a first aspect provides a gas sensor as set out in claim 1.

According to the present invention in this first aspect, at first, the oxygen, which is contained in the measurement gas introduced from the external space, is pumping-processed by the main pumping means, and the oxygen is adjusted to have a predetermined concentration. The measurement gas, which has been adjusted for the concentration of oxygen by means of the main pumping means, is introduced into the measuring pumping means in the next step. The measuring pumping means pumping-processes the oxygen contained in the measurement gas on the basis of the measuring voltage applied between the pair of detecting electrodes. The pumping current, which is generated in the measuring pumping means depending on the amount of oxygen pumping-processed by the measuring pumping means, is detected by the current-detecting means. Thus, the predetermined gas component corresponding to the amount of oxygen is measured.

That is, the measuring pumping means is operated while applying a voltage sufficient to decompose the predetermined gas component, between the pair of detecting electrodes, or the measuring pumping means is arranged with a decomposing catalyst for decomposing the predetermined gas component. By doing so, it is possible to pumping-process the oxygen produced from the predetermined gas component decomposed by the action of the voltage and/or the decomposing catalyst. The pumping current generated thereby is detected by the current-detecting means. Thus, the predetermined gas component is measured corresponding to the amount of oxygen.

According to second aspect of the present invention, there is provided a gas sensor as set out in claim 3.

According to the present invention in this second aspect, at first, the oxygen, which is contained in the measurement gas introduced from the external space, is pumping-processed by the main pumping means, and the oxygen is adjusted to have a predetermined concentration. The measurement gas, which has been adjusted for the concentration of oxygen by means of the main pumping means, is introduced into the concentration-detecting means in the next step. The concentration-detecting means generates the electromotive force of the oxygen concentration cell corresponding to the difference between the amount of oxygen contained in the measurement gas after being pumping-processed by the main pumping means and the amount of oxygen contained in the gas existing on the side of the other detecting electrode. The electromotive force is detected by the voltage-detecting means Thus, the predetermined gas component corresponding to the amount of oxygen is measured.

In this aspect, when the concentration-detecting means is arranged with a decomposing catalyst for decomposing the predetermined gas component, the electromotive force of the oxygen concentration cell is generated between the pair of detecting electrodes, which corresponds to a difference between the amount of oxygen produced from the predetermined gas component decomposed by the action of the decomposing catalyst and the amount of oxygen contained in the gas existing on the side of the other detecting electrode. The electromotive force is detected by the voltage-detecting means. Thus, the predetermined gas component corresponding to the amount of oxygen is measured.

Explanation will now be made referring to, for example, a gas sensor for measuring NO. The sensitivity to NO of the gas sensor is increased as the $O_2$ concentration becomes high. It has been revealed that the increase in sensitivity relates not only to the shortage of $O_2$ pumping but also to the reaction of $NO + (1/2)O_2 \rightarrow O_2$, effected on the one of the pumping electrodes exposed to the process space for the measurement gas introduced from the external space.

The reaction of $NO + (1/2)O_2 \rightarrow NO_2$ described above results from the fact that the oxidizing catalyst performance remains in the one of the pumping electrodes. Therefore, it is possible to suppress the reaction of $NO + (1/2)O_2 \rightarrow NO_2$

by further lowering the catalytic activity of the one of the pumping electrodes.

When the gas sensor is used (operated) for a long period of time, then the electrode is subjected to an aging process, and the catalytic activity is increased. When the catalytic activity of the electrode for adjusting the $O_2$ concentration is increased, the decomposition of NO occurs in the process space for the main pumping means or in the process space for the measuring pumping means or the concentration-detecting means, resulting in occurrence of decrease in sensitivity (degree of decrease in the pumping current flowing between the pair of detecting electrodes, or in the electromotive force generated between the pair of detecting electrodes).

The cause of expression of the catalytic activity resides in the decrease in Au concentration in the electrode when the alloy composed of Au and the metal belonging to the platinum group is used for the electrode. The route of diffusion of Au includes (1) evaporation of Au and (2) diffusion of Au to lead wires. Therefore, if the amount of Au added to the electrode is increased, the catalytic activity is not expressed even when Au in the electrode is decreased as a result of operation of the gas sensor for a long period of time.

In the present invention described above, at least one of the electrodes exposed to the process space for the measurement gas introduced from the external space is the cermet electrode composed of the alloy of Au and the metal of the platinum group and the ceramic component, and Au is contained in the alloy in a ratio of not less than 0.3 wt% and not more than 35 wt%.

In the present invention, the electrode containing the alloy has an extremely low activity as the NO-decomposing catalyst, and it does not decompose NO even at a low partial pressure of oxygen. That is, the reaction of NO + (1/2) $O_2 \rightarrow NO_2$ is suppressed on the one of the pumping electrodes exposed to the process space for the main pumping means, and the NO sensitivity of the gas sensor scarcely depends on the concentration of $O_2$ contained in the measurement gas.

The amount of Au to be added is defined to be not less than 0.3 wt% and not more than 35 wt%. Accordingly, even when the Au component in the one of the pumping electrodes of the main pumping means is decreased due to the operation of the gas sensor for a long period of time, the catalytic activity of the one of the pumping electrodes is not expressed.

As a result, in the gas sensor according to the present invention, the oxygen, which behaves as a disturbing component for the measurement of the predetermined gas component, can be excluded to be substantially zero without exerting any influence on the measurement of the predetermined gas component. Accordingly, it is possible to measure the predetermined gas component contained in the measurement gas highly accurately and stably for a long period of time by the aid of the measuring pumping means and the current-detecting means.

It is preferable that Au is contained in the alloy of Au and the metal of the platinum group in an amount of not less than 0.5 wt% and not more than 10 wt% with respect to the metal of the platinum group.

It is also preferable that the gas sensor constructed as described above further comprises a concentration-measuring means including a pair of measuring electrodes one of which is disposed on a side for introducing the measurement gas from the external space thereinto, for measuring an electromotive force generated depending on a difference between an amount of oxygen contained in the measurement gas during the pumping process effected by the main pumping means and an amount of oxygen contained in an atmosphere on a side of the other measuring electrode; and a main pumping control means for adjusting the control voltage for the main pumping means on the basis of the electromotive force detected by the concentration-measuring means.

Accordingly, the concentration-measuring means generates the electromotive force corresponding to the difference between the amount of oxygen contained in the measurement gas during the pumping process effected by the main pumping means and the amount of oxygen contained in the gas existing on the side of the other measuring electrode. The level of the control voltage, which is applied between the pair of pumping electrodes of the main pumping means, is adjusted on the basis of the electromotive force by the aid of the main pumping control means.

The main pumping means pumping-processes the oxygen contained in the measurement gas introduced from the external space, in an amount corresponding to the level of the control voltage. The concentration of oxygen in the measurement gas is subjected to feedback control to achieve a predetermined level, in accordance with the supply of the level-adjusted control voltage to the main pumping means.

It is preferable that the main pumping control means is provided with a comparing means for determining a deviation between the electromotive force and a comparative voltage, and the level of the control voltage is adjusted on the basis of the deviation obtained by the comparing means. In this embodiment, the control voltage is subjected to feedback control so that the terminal voltage is converged to the comparative voltage.

It is preferable that the gas sensor constructed as described above further comprises a pumping current-detecting means for detecting a pumping current flowing through the main pumping means during the pumping process effected by the main pumping means, and a comparative voltage-correcting means for correcting a level of the comparative voltage on the basis of a value of the pumping current detected by the pumping current-detecting means.

Usually, the current flows through the main pump during the pumping process effected by the main pumping means. Accordingly, the amount corresponding to the voltage drop caused by the impedance of the main pumping means appears as an error in the adjustment for the level of the control voltage.

In this invention, the pumping current flowing through the main pumping means is detected, and an obtained pumping current value is reflected to the comparative voltage. Therefore, the error is effectively absorbed, making it possible to accurately perform the feedback control without causing any oscillation phenomenon in the feedback control for the main pumping means.

It is also preferable for the gas sensor constructed as described above that the one pumping electrode of the main pumping means is commonly used as the one measuring electrode of the concentration-measuring means. In this embodiment, the one pumping electrode is commonly provided and used as the one measuring electrode. Accordingly, when the oxygen concentration in the process space is changed in accordance with the pumping process effected by the main pumping means, the electromotive force measured by the concentration-measuring means is also changed without any time delay. Therefore, it is possible to perform the feedback control effected by the concentration-measuring means for the main pumping means without causing any oscillation.

When the one pumping electrode of the main pumping means is commonly used as the one measuring electrode of the concentration-measuring means as described above, if the oxygen concentration in the measurement gas introduced from the external space is high, then an excessive correction state is given, in which the decomposition of NO takes place in the process space for the main pumping means, resulting in decrease in sensitivity.

However, in the present invention, the one pumping electrode of the main pumping means is selected as at least one electrode exposed to the process space for the measurement gas introduced from the external space, making it possible that the one pumping electrode is the cermet electrode composed of the alloy of Au and the metal of the platinum group and the ceramic component, and Au is contained in the alloy in the ratio of not less than 0.3 wt% and not more than 35 wt%. Accordingly, even when the excessive correction state is given as described above, the decomposition of NO is suppressed in the process space for the main pumping means, making it possible to effectively avoid the decrease in sensitivity.

The gas sensor of the invention in the first and second aspects constructed as described above further comprises an auxiliary pumping means including a pair of auxiliary pumping electrodes one of which is formed in the vicinity of the one detecting electrode, for pumping-processing oxygen contained in the measurement gas after being pumping-processed by the main pumping means on the basis of an auxiliary pumping voltage applied between the pair of auxiliary pumping electrodes.

Accordingly, the measurement gas, which has been firstly subjected to coarse adjustment for the oxygen concentration to have a predetermined concentration by the aid of the main pumping means, is further subjected to fine adjustment for the oxygen concentration by the aid of the auxiliary pumping means.

In general, when the oxygen concentration in the measurement gas in the external space is greatly changed (for example, from 0 % to 20 %), then the oxygen concentration distribution in the measurement gas to be introduced into the main pumping means is greatly changed, and the amount of oxygen to be introduced into the measuring pumping means or the concentration-detecting means is also changed.

During this process, the oxygen concentration in the measurement gas after being pumping-processed by the main pumping means is finely adjusted in accordance with the pumping process effected by the auxiliary pumping means. However, owing to the pumping process performed by the main pumping means, the change in concentration of oxygen in the measurement gas introduced into the auxiliary pumping means is greatly reduced as compared with the change in concentration of oxygen in the measurement gas introduced from the external space (measurement gas introduced into the main pumping means). Accordingly, it is possible to accurately and constantly control the oxygen concentration in the vicinity of the one detecting electrode of the measuring pumping means or in the vicinity of the one detecting electrode of the concentration-detecting means.

Therefore, the concentration of the predetermined gas component introduced into the measuring pumping means or the concentration-detecting means is scarcely affected by the change in concentration of oxygen in the measurement gas (measurement gas introduced into the main pumping means). As a result, the pumping current value detected by the current-detecting means or the electromotive force detected by the voltage-detecting means is not affected by the change in oxygen concentration in the measurement gas, which has a value accurately corresponding to the amount of the objective component existing in the measurement gas.

In the invention described above, it is also preferable that the other auxiliary pumping electrode is commonly used as the outer pumping electrode of the main pumping means. Accordingly, the oxygen in the process space for the auxiliary pumping means is subjected to the pumping process to be pumped out to the external space by the aid of the outer pumping electrode.

It is also preferable that the gas sensor constructed as described above further comprises an auxiliary concentration-measuring means including a pair of auxiliary measuring electrodes one of which is disposed on a side for introducing the measurement gas after being pumping-processed by the main pumping means, for generating an electromotive force corresponding to a difference between an amount of oxygen contained in the measurement gas after being pumping-processed by the main pumping means and an amount of oxygen contained in a gas existing on a side of the other auxiliary measuring electrode, and an auxiliary pumping control means for adjusting the auxiliary pumping voltage for the auxiliary pumping means on the basis of the electromotive force detected by the auxiliary concentration-measuring means.

Accordingly, the auxiliary concentration-measuring means generates the electromotive force corresponding to the difference between the amount of oxygen contained in the measurement gas during the pumping process effected by the auxiliary pumping means and the amount of oxygen contained in the gas existing on the side of the other measuring electrode. The level of the auxiliary pumping voltage applied to the pair of auxiliary pumping electrodes of the auxiliary pumping means is adjusted on the basis of the electromotive force by the aid of the auxiliary pumping control means.

The auxiliary pumping means pumping-processes oxygen contained in the measurement gas introduced from the external space, in an amount corresponding to the level of the auxiliary pumping voltage. The concentration of oxygen in the measurement gas is subjected to feedback control to be at a predetermined level in accordance with the supply of the level-adjusted auxiliary pumping voltage to the auxiliary pumping means.

In the gas sensor constructed as described above, it is also preferable that the one auxiliary measuring electrode is commonly used as the one auxiliary pumping electrode of the auxiliary pumping means, and the other auxiliary measuring electrode is commonly used as the other detecting electrode of the concentration-detecting means.

In this embodiment, when the oxygen concentration in the process space is changed in accordance with the pumping process effected by the auxiliary pumping means, the electromotive force measured by the auxiliary concentration-measuring means is also changed without any time delay. Accordingly, it is possible to appropriately perform the feedback control effected by the auxiliary concentration-measuring means for the auxiliary pumping means without causing any oscillation.

Preferably, the gas sensor is constructed such that the plurality of electrodes for constructing the respective means are formed on a substrate including solid electrolytes, and the substrate is constructed by forming an insulative layer and an electrode layer on a green sheet composed of the solid electrolyte, stacking and integrating a plurality of the green sheets into one stacked unit, and sintering the stacked unit.

For example, an electrode paste is printed on the green sheet, and the respective sheets are stacked, cut, and integrally sintered to produce the gas sensor. In this procedure, when at least one electrode exposed to the process space for the measurement gas introduced from the external space is constructed by a cermet electrode composed of an alloy of Au and a metal of the platinum group and a ceramic component, if Au is added in an amount of not less than 37 wt%, then abnormal grain growth occurs in the Au-Pt alloy. As a result, for example, it become difficult to perform the sintering at 1300°C. That is, such an electrode cannot be sintered in an integrated manner. Therefore, the upper limit of the adding amount of Au is less than 35 wt%.

On the other hand, if Au is added in an amount of 0.2 wt%, the detection sensitivity to the predetermined gas component is lowered in the measuring pumping means and the concentration-detecting means in the excessive correction state described above. Further, the detection sensitivity is also lowered in the measuring pumping means and the concentration-detecting means when the gas sensor is operated for a long period of time.

The above and other objects, features, and advantages of the present invention will become more apparent from the following description when taken in conjunction with the accompanying drawings in which a preferred embodiment of the present invention is shown by way of illustrative example.

## DESCRIPTION OF THE DRAWINGS

FIG. 1 shows a cross-sectional view illustrating a gas sensor according to a first embodiment.

FIG. 2 shows characteristic curves illustrating the limiting current characteristic of the gas sensor according to the first embodiment.

FIG. 3 shows characteristic curves illustrating experimental results obtained in a first illustrative experiment (relationship between the NO concentration and the pumping current Ip2).

FIG. 4 shows characteristic curves illustrating experimental results obtained in a second illustrative experiment (relationship between the Au-adding amount and the NO decomposition ratio, and relationship between the Au-adding amount and the NO oxidation ratio).

FIG. 5 shows characteristic curves illustrating experimental results obtained in a third illustrative experiment (relationship between the Au-adding amount and the NO decomposition ability).

FIG. 6 shows characteristic curves illustrating experimental results obtained in a fourth illustrative experiment (degree of decrease in sensitivity in the excessive correction state).

FIG. 7 shows characteristic curves illustrating experimental results obtained in a fifth illustrative experiment (relationship between the durable time and the sensitivity to NO).

FIG. 8 shows a cross-sectional view illustrating a gas sensor according to a second embodiment.

FIG. 9 shows characteristic curves illustrating experimental results obtained in an illustrative experiment concerning the gas sensor according to the second embodiment (relationship between the NO concentration and the pumping current Ip2).

FIG. 10 shows characteristics illustrating the relationship between the Au-adding amount and the presence or absence of the occurrence of abnormal grain growth, and the change in electrode resistance.

FIG. 11 shows a cross-sectional view illustrating the gas analyzer concerning the conventional technique.

DESCRIPTION OF THE PREFERRED EMBODIMENTS

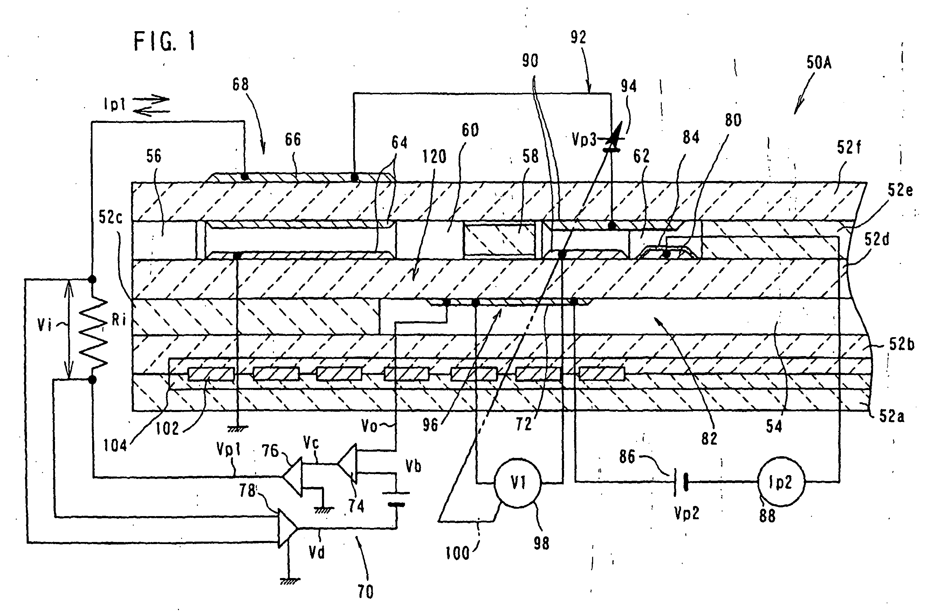

Explanation will be made below with reference to FIGs. 1 to 10 for several illustrative embodiments in which the gas sensor according to the present invention is applied to gas sensors for measuring oxides such as NO, $NO_2$, $SO_2$, $CO_2$, and $H_2O$ contained in, for example, atmospheric air and exhaust gas discharged from vehicles or automobiles, and inflammable gases such as CO and CnHm.

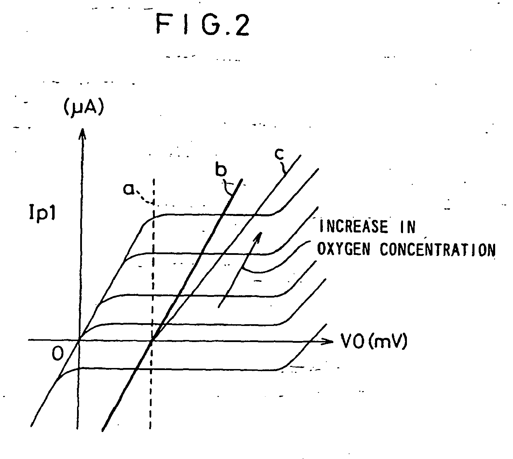

At first, as shown in FIGs. 1 and 2, a gas sensor 50A according to the first embodiment is generally constructed to have a lengthy plate-shaped configuration comprising, for example, six stacked solid electrolyte layers 52a to 52f composed of ceramics based on the use of oxygen ion-conductive solid electrolytes such as $ZrO_2$. First and second layers from the bottom are designated as first and second substrate layers 52a, 52b respectively. Third and fifth layers from the bottom are designated as first and second spacer layers 52c, 52e respectively. Fourth and sixth layers from the bottom are designated as first and second solid electrolyte layers 52d, 52f respectively.

Specifically, the first spacer layer 52c is stacked on the second substrate layer 52b. The first solid electrolyte layer 52d, the second spacer layer 52e, and the second solid electrolyte layer 52f are successively stacked on the first spacer layer 52c.

A space (reference gas-introducing space) 54, into which a reference gas such as atmospheric air to be used as a reference for measuring oxides is introduced, is formed between the second substrate layer 52b and the first solid electrolyte layer 52d, the space 54 being comparted by a lower surface of the first solid electrolyte layer 52d, an upper surface of the second substrate layer 52b, and side surfaces of the first spacer layer 52c.

The second spacer layer 52e is interposed between the first and second solid electrolyte layers 52d, 52f. First and second diffusion rate-determining sections 56, 58 are also interposed between the first and second solid electrolyte layers 52d, 52f.

A first chamber 60 for adjusting the partial pressure of oxygen in a measurement gas is formed and comparted by a lower surface of the second solid electrolyte layer 52f, side surfaces of the first and second diffusion rate-determining sections 56, 58, and an upper surface of the first solid electrolyte layer 52d. A second chamber 62 for finely adjusting the partial pressure of oxygen in the measurement gas and measuring nitrogen oxides (for example, NOx) in the measurement gas is formed and comparted by a lower surface of the second solid electrolyte layer 52f, a side surface of the second diffusion rate-determining section 58, a side surface of the second spacer layer 52e, and an upper surface of the first solid electrolyte layer 52d.

The external space communicates with the first chamber 60 via the first diffusion-rate determining section 56, and the first chamber 60 communicates with the second chamber 62 via the second diffusion rate-determining section 58.

The first and second diffusion-rate determining sections 56, 58 give predetermined diffusion resistances to the measurement gas to be introduced into the first and second chambers 60, 62 respectively. Each of the first and second diffusion-rate determining sections 56, 58 can be formed as a passage composed of, for example, a porous material, or a small hole having a predetermined cross-sectional area so that the measurement gas may be introduced.

Especially, the second diffusion-rate determining section 58 is arranged and filled with a porous material comprising, for example, $ZrO_2$. The diffusion resistance of the second diffusion-rate determining section 58 is made larger than the diffusion resistance of the first diffusion-rate determining section 56.

The atmosphere in the first chamber 60 is introduced into the second chamber 62 under the predetermined diffusion resistance via the second diffusion rate-determining section 58.

An inner pumping electrode 64 composed of a porous cermet electrode is formed continuously over an upper surface portion for forming the first chamber 60, of the upper surface of the first solid electrolyte layer 52d, the side surface of the second spacer layer 52e, and the lower surface of the second solid electrolyte layer 52f. An outer pumping electrode 66 is formed on a portion corresponding to the inner pumping electrode 64, of the upper surface of the second solid electrolyte layer 52f. An electrochemical pumping cell, i.e., a main pumping cell 68 is constructed by the inner pumping electrode 64, the outer pumping electrode 66, and the second solid electrolyte layer 52f interposed between the both electrodes 64, 66.

A desired control voltage (pumping voltage) Vp1 is applied between the inner pumping electrode 64 and the outer pumping electrode 66 of the main pumping cell 68 by the aid of a feedback control system 70 described later on to allow a pumping current Ip1 to flow in a positive or negative direction between the outer pumping electrode 66 and the inner pumping electrode 64. Thus, the oxygen in the atmosphere in the first chamber 60 can be pumped out to the external space, or the oxygen in the external space can be pumped into the first chamber 60.

A reference electrode 72 for measuring the partial pressure of oxygen of the measurement gas is formed on a lower surface portion for forming the reference gas-introducing space 54, of the lower surface of the first solid electrolyte layer 52d.

In this arrangement, an electromotive force of an oxygen concentration cell is generated on the basis of a difference between a partial pressure of oxygen in the atmospheric air introduced into the reference gas-introducing space 54 and a partial pressure of oxygen in the measurement gas introduced into the first chamber 60. The electromotive force is represented by an electric potential difference V0 between the reference gas-introducing space 54 and the first chamber 60. The electric potential difference V0 can be determined in accordance with the following Nernst's equation.

$$VO = RT/4F \cdot ln(P1(O_2)/PO(O_2))$$

R: gas constant;
T: absolute temperature;
F: Faraday constant;
$P1(O_2)$: partial pressure of oxygen in the gas-introducing space;
$PO(O_2)$: partial pressure of oxygen in the reference gas.

Therefore, the partial pressure of oxygen in the first chamber 60 can be measured by measuring the electric potential difference V0 generated on the basis of the Nernst's equation. That is, an electrochemical sensor cell, i.e., a controlling oxygen partial pressure-detecting cell 120 is constructed by the inner pumping electrode 64, the reference electrode 72, and the first solid electrolyte layer 52d.

In general, a pumping voltage Vp1, which is set on the basis of the electric potential difference V0, is applied between the inner pumping electrode 64 and the outer pumping electrode 66 by the aid of feedback control system 70. Oxygen is pumped out from or pumped in into the first chamber 60 by the main pumping cell 68 in accordance with application of the pumping voltage Vp1. Accordingly, the partial pressure of oxygen in the first chamber 60 is set to have a predetermined value.

The gas sensor 50A according to the first embodiment is constructed such that the electromotive force (voltage) V0 generated between the inner pumping electrode 64 and the reference electrode 72 is measured to determine a difference between the measured voltage V0 and the reference voltage Vb so that the pumping voltage Vp1 is controlled on the basis of the determined differential voltage Vc by the aid of the feedback control system 70.

Explanation will now be made for an exemplary circuit for the feedback control system 70 which is wired and connected as follows. That is, the feedback control system 70 is provided with a comparator 74 for comparing the reference voltage Vb with the terminal voltage V0 between the reference electrode 72 and the inner pumping electrode 64 and outputting an output in an amount corresponding to a difference therebetween as a voltage signal Vc, and a first amplifier 76 for amplifying the output Vc from the comparator 74 with a predetermined gain. The output voltage (differential voltage) from the first amplifier 76 is applied, as the pumping voltage Vp1 to be supplied to the main pumping cell 68, between the inner pumping electrode 64 and the outer pumping electrode 66. In this embodiment, the inner pumping electrode 64 is grounded.

In this arrangement, when the amount of oxygen pumped out by the main pumping cell 68 is changed, and the oxygen concentration in the first chamber 60 is changed, then the terminal voltage V0 between the inner pumping electrode 64 of the main pumping cell 68 and the reference electrode 72 is changed without any time delay (the terminal voltage is changed in real-time). Accordingly, the oscillation phenomenon in the feedback control system 70 can be effectively suppressed.

In the feedback control system 70, the pumping voltage Vp1 (output voltage) is subjected to feedback control so that the terminal voltage V0 between the inner pumping electrode 64 and the reference electrode 72 converges to the same level as that of the reference voltage Vb.

In addition to the arrangement described above, the feedback control system 70 further comprises a resistor Ri for detecting the pumping current, the resistor Ri being inserted and connected between the outer pumping electrode 66 and the output end of the first amplifier 76, and a second amplifier 78 connected for amplifying a terminal voltage Vi of the resistor Ri with a predetermined gain and superimposing an obtained amplified voltage Vd on the reference voltage Vb.

That is, the feedback control system 70 is wired and connected such that the current, which flows between the inner pumping electrode 64 and the outer pumping electrode 66 corresponding to the oxygen pumped out and pumped in by main pumping cell 68, is converted into a voltage Vi corresponding to a value of the current in accordance with the voltage drop effectuated in the resistor Ri, and the voltage Vi is supplied to the second amplifier 78.

In general, the current (pumping current) Ip1 flows through the main pumping cell 68 when the oxygen is pumped out and pumped in by the aid of the main pumping cell 68. Therefore, the amount corresponding to the voltage drop resulting from the impedance of the main pumping cell 68 appears as an error in the operation of level adjustment for the pumping voltage Vp1.

However, in the gas sensor 50A according to the first embodiment, the pumping current Ip1 flowing through the main pumping cell 68 is converted into the voltage Vi by using the resistor Ri, and the voltage Vi is amplified by the second amplifier 78 with the predetermined gain to obtain the correction voltage Vd which is superimposed on the reference voltage Vb. That is, only the amount corresponding to the voltage drop resulting from the boundary resistance (impedance) of the inner pumping electrode 64 is superimposed on the voltage V0 between the inner pumping electrode 64 and the reference electrode 72. The amount corresponding to the voltage drop is considerably decreased. Therefore, it is sufficient for the amount corresponding to the voltage drop to be slightly corrected, and hence the accuracy is improved to that extent. In other words, the amount corresponding to the voltage drop resulting from the impedance of the main pumping cell 68 is reflected, as the correction voltage Vd, to the reference voltage Vb (or superimposed on the reference voltage Vb). Therefore, the control effected by the feedback control system 70 is corrected so that the pumping current Ip1 flowing through the main pumping cell 68 is constant. Accordingly, it is possible to effectively absorb the error resulting from the impedance of the main pumping cell 68 with respect to the pumping voltage Vp1, making it possible to perform the feedback control for the pumping voltage Vp1 with a high degree of accuracy.

The process described above results in a successful operation of the gas sensor 50A in which the operation point thereof follows points corresponding to an identical amount of the electromotive force as shown in FIG. 2 (indicated by a straight line "b" shown in FIG. 2). Thus, the oxygen concentration in the first chamber 60 can be detected highly accurately. In FIG. 2, the horizontal axis indicates the voltage V0 between the inner pumping electrode 64 and the reference electrode 72, and the vertical axis indicates the pumping electrode Ip1 flowing through the main pumping cell 68. A straight line "a" indicates a state of constant control in which no correction is made for the feedback control system 70. A straight line "c" indicates an excessive correction state which appears when the oxygen concentration is changed in a great degree when correction is made for the feedback control system 70. The excessive correction state will be described later on.

In the gas sensor 50A according to the first embodiment, a detecting electrode 80 having a substantially rectangular planar configuration and composed of a porous cermet electrode is formed at a portion separated from the second diffusion rate-determining section 58, on an upper surface portion for forming the second chamber 62, of the upper surface of the first solid electrolyte layer 52d. An electrochemical pumping cell, i.e., a measuring pumping cell 82 is constructed by the detecting electrode 80, the reference electrode 72, and the first solid electrolyte layer 52d.

A porous $Al_2O_3$ layer or a porous $ZrO_2$ layer for forming a third diffusion rate-determining section 84 is formed so as to surround the detecting electrode 80 of the measuring pumping cell 82. A third chamber is formed by a boundary between the third diffusion rate-determining section 84 and the detecting electrode 80.

The detecting electrode 80 may be appropriately constructed by selecting a nitrogen oxide-decomposing catalyst, for example, an Rh cermet, a material having a low catalytic activity, or a nitrogen oxide-decomposing catalyst arranged in the vicinity of a material having a low catalytic activity. In the embodiment of the present invention, the detecting electrode 80 is composed of a porous cermet comprising Rh as a metal capable of reducing NOx as the objective gas component and zirconia as a ceramic.

Accordingly, the detecting electrode 80 functions as an NOx-reducing catalyst for reducing NOx existing in the atmosphere in the second chamber 62. Further, the oxygen in the atmosphere in the second chamber 62 can be pumped out to the reference gas-introducing space 54 by applying a constant voltage Vp2 between the detecting electrode 80 and the reference electrode 72 by the aid of a DC power source 86. The pumping current Ip2, which is allowed to flow in accordance with the pumping operation performed by the measuring pumping cell 82, is detected by an ammeter 88.

On the other hand, an auxiliary pumping electrode 90 composed of a porous cermet electrode is continuously formed over an upper surface portion for forming the second chamber 62 (portion except for the region for forming the detecting electrode 80), of the first solid electrolyte layer 52d, side surfaces of the second spacer layer 52e, and the entire lower surface of the second solid electrolyte layer 52f. An electrochemical pumping cell, i.e., an auxiliary pumping cell 92 is constructed by the auxiliary pumping electrode 90, the outer pumping electrode 66 of the main pumping cell 68, and the second solid electrolyte layer 52f interposed between the both electrodes 66, 90.

An auxiliary control voltage Vp3 is applied between the outer pumping electrode 66 and the auxiliary pumping electrode 90 of the auxiliary pumping cell 92 by the aid of a variable power source 94. Thus, the oxygen in the atmosphere in the second chamber 62 can be pumped out to the external space.

In the gas sensor 50A according to the first embodiment, an auxiliary oxygen partial pressure-detecting cell 96 is constructed by the auxiliary pumping electrode 90, the reference electrode 72, and the first solid electrolyte layer 52d.

An electromotive force V1 is generated between the auxiliary pumping electrode 90 and the reference electrode 72 on the basis of a difference in oxygen concentration between the atmosphere in the second chamber 62 and the reference gas (atmospheric air) in the reference gas-introducing space 54. The auxiliary oxygen partial pressure-detecting cell 96 makes it possible to detect the partial pressure of oxygen in the atmosphere in the second chamber 62 by measuring the generated electromotive force V1 by means of a voltmeter 98.

The value of the partial pressure of oxygen detected as described above is used to control the auxiliary pumping voltage Vp3 of the variable power source 94 to be constant by the aid of a feedback control system 100. The partial pressure of oxygen in the atmosphere in the second chamber 62 is controlled to have a low value of partial pressure of oxygen which does not substantially affects the measurement for the amount of the objective component under a condition in which the objective gas component (NOx) is not substantially reduced or decomposed.

Specifically, the variable power source 94 is controlled to provide a voltage value, for example, 450 mV of a magnitude to give a limiting current to the pumping for the oxygen produced during the decomposition effected in the auxiliary pumping cell 92. In this embodiment, when the amount of oxygen pumped out by the auxiliary pumping cell 92 is changed, and the oxygen concentration in the atmosphere in the second chamber 62 is changed, then the terminal voltage V1 between the auxiliary pumping electrode 90 and the reference electrode 72 is changed without any time delay. Accordingly, no oscillation phenomenon occurs in the feedback control system 100 for the variable power source 94. Thus, it is possible to highly accurately control the oxygen concentration in the second chamber 62.

When it is intended to control the partial pressure of oxygen in the atmosphere in the first chamber 60 to have a low value of the partial pressure of oxygen which does not substantially affect the measurement of NOx, by operating the main pumping cell 68, in other words, when the pumping voltage Vp1 is adjusted by the aid of the feedback control system 70 so that the electromotive force V0 generated between the inner pumping electrode 64 and the reference electrode 72 is constant, if the oxygen concentration in the measurement gas greatly changes, for example, in a range of 0 to 20 %, then the respective partial pressures of oxygen in the atmosphere in the second chamber 62 and in the atmosphere in the vicinity of the detecting electrode 80 slightly change in ordinary cases. This phenomenon is caused probably because of the following reason. That is, when the oxygen concentration in the measurement gas increases, the distribution of the oxygen concentration occurs in the widthwise direction and the thickness direction in the first chamber 60. The distribution of the oxygen concentration changes depending on the oxygen concentration in the measurement gas.

However, in the case of the gas sensor 50A according to the first embodiment, the auxiliary pumping cell 92 is provided for the second chamber 62 so that the partial pressure of oxygen in its internal atmosphere always has a constant low value of the partial pressure of oxygen. Accordingly, even when the partial pressure of oxygen in the atmosphere introduced from the first chamber 60 into the second chamber 62 changes depending on the oxygen concentration in the measurement gas, the partial pressure of oxygen in the atmosphere in the second chamber 62 can be always made to have a constant low value, owing to the pumping operation performed by the auxiliary pumping cell 92. As a result, the partial pressure of oxygen can be controlled to have a low value at which the measurement of NOx is not substantially affected.

NOx in the measurement gas introduced into the detecting electrode 80 is reduced or decomposed around the detecting electrode 80. Thus, for example, a reaction of $NO \rightarrow 1/2N_2 + 1/2O_2$ is allowed to occur. In this process, a predetermined voltage Vp2, for example, 400 mV (700°C) is applied between the detecting electrode 80 and the reference electrode 72 for constructing the measuring pumping cell 82, in a direction to pump out the oxygen from the second chamber 62 to the reference gas-introducing space 54.

Therefore, the pumping current Ip2 flowing through the measuring pumping cell 82 has a value which is proportional to a sum of the oxygen concentration in the atmosphere introduced into the second chamber 62, i.e., the oxygen concentration in the second chamber 62 and the oxygen concentration produced by reduction or decomposition of NOx by the aid of the detecting electrode 80.

The oxygen concentration in the atmosphere in the second chamber 62 is controlled to be constant by means of the auxiliary pumping cell 92. Accordingly, the pumping current Ip2 flowing through the measuring pumping cell 82 is proportional to the NOx concentration. In this embodiment, the third diffusion rate-determining section 84 is formed to cover the detecting electrode 80. Accordingly, the NOx concentration corresponds to the amount of diffusion of NOx limited by the third diffusion rate-determining section 84. Therefore, even when the oxygen concentration in the measurement gas greatly changes, it is possible to accurately measure the NOx concentration, based on the use of the measuring pumping cell 82 by the aid of the ammeter 88. That is, the pumping current value Ip2 of the measuring pumping cell 82 represents the amount obtained by reduction or decomposition of almost all NO. Therefore, the pumping current value Ip2 does not depend on the oxygen concentration in the measurement gas.

The gas sensor 50A according to the first embodiment further comprises a heater 102 for generating heat in accordance with electric power supply from the outside. The heater 102 is embedded in a form of being vertically interposed between the first and second substrate layers 52a, 52b. The heater 102 is provided in order to increase the conductivity of oxygen ion. An insulative layer 104 composed of alumina or the like is formed to cover upper and lower surfaces of the heater 102 so that the heater 102 is electrically insulated from the first and second substrate layers 52a, 52b.

As shown in Fig. 1, the heater 102 is arranged over the entire portion ranging from the first chamber 60 to the second chamber 62. Accordingly, each of the first chamber 60 and the second chamber 62 is heated to a predetermined temperature. Simultaneously, each of the main pumping cell 68, the measuring pumping cell 82, and the auxiliary pumping cell 92 is also heated to a predetermined temperature and maintained at that temperature.

Next, explanation will be made for the composition of each of the electrodes included in the gas sensor 50A according to the first embodiment.

Each of the inner pumping electrode 64 and the outer pumping electrode 66 for constructing the main pumping cell 68, the detecting electrode 80 and the reference electrode 72 for constructing measuring pumping cell 82, and the auxiliary pumping electrode 90 for constructing the auxiliary pumping cell 92 is composed of an inactive material having a low catalytic activity on NOx, for example, NO contained in the measurement gas introduced into the gas sensor 50A.

For example, both of the outer pumping electrode 66 which contacts with the external space, and the reference electrode 72 which contacts with the reference gas can be constructed by Pt electrodes.

The detecting electrode 80 can be constructed, for example, by an Rh cermet electrode which functions as an NOx-decomposing catalyst.

On the other hand, the inner pumping electrode 64 which contacts with the measurement gas in the first chamber 60, and the auxiliary pumping electrode 90 which contacts with the measurement gas in the second chamber 62 can be constructed by porous cermet electrodes. In this embodiment, each of them is composed of a metal such as Pt and a ceramic such as $ZrO_2$. It is necessary to use a material having a weak reducing ability or no reducing ability with respect to the NOx component in the measurement gas, for the inner pumping electrode 64 and the auxiliary pumping electrode 90 disposed at the inside of the first chamber 60 and the second chamber 62 to make contact with the measurement gas. Each of these electrodes is composed of, for example, a compound having the perovskite structure such as $La_3CuO_4$, a cermet comprising a ceramic and a metal having a low catalytic activity such as Au, or a cermet comprising a ceramic, a metal of the Pt group, and a metal having a low catalytic activity such as Au.

In this embodiment, each of the inner pumping electrode 64 and the auxiliary pumping electrode 90 is composed of a cermet electrode comprising an alloy of Au and a metal of the platinum group and a ceramic component such as $ZrO_2$. Moreover, Au is contained in the alloy in a ratio of not less than 0.3 wt% and not more than 35 wt%.

Therefore, the inner pumping electrode 64 and the auxiliary pumping electrode 90 containing the alloy have extremely low activities as NOx-decomposing catalysts, which do not decompose NOx even at a low partial pressure of oxygen. The reaction of NO + $(1/2)O_2 \rightarrow NO_2$ is suppressed on the inner pumping electrode 64 in the process space for the main pumping cell 68. Therefore, the sensitivity to NO of the gas sensor 50A scarcely depends on the concentration of $O_2$ contained in the measurement gas.

Further, the Au-adding amount is defined to be not less than 0.3 wt% and not more than 35 wt%. Therefore, even when the Au component in the inner pumping electrode 64 of the main pumping cell 68 is decreased due to the operation of the gas sensor 50A for a long period of time, the catalytic activity of the inner pumping electrode 64 is not expressed.

As a result, in the gas sensor 50A according to the first embodiment described above, the oxygen, which behaves as a disturbing component for the measurement of NOx, can be excluded to be substantially zero without exerting any influence on the measurement of NOx. Accordingly, it is possible to measure NOx contained in the measurement gas highly accurately and stably for a long period of time by the aid of the measuring pumping cell 82.

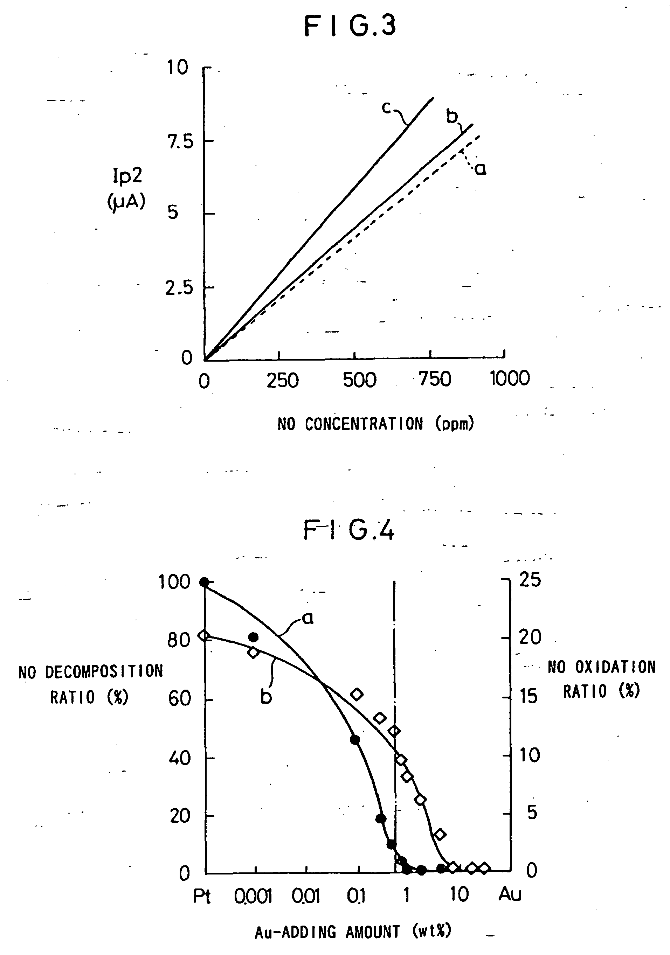

Explanation will now be made for the ground to define the Au-adding amount to be not less than 0.3 wt% and not more than 35 wt%, while referring to five illustrative experiments shown in FIGs. 3 to 7.

At first, in the first illustrative experiment, there were prepared a gas sensor constructed in the same manner as the gas sensor 50A according to the first embodiment in which Au was added to the alloy for constructing the inner pumping electrode 64 in an amount of 0.3 wt% (Example 1), and a gas sensor constructed in the same manner as the gas sensor 50A according to the first embodiment in which Au was added in an amount of 0.1 wt% (Comparative Example 1). Observation was made for whether or not the reaction of NO + $(1/2)O_2 \rightarrow NO_2$ was suppressed by increasing the Au-adding amount.

Experimental results are shown in FIG. 3. In FIG. 3, a characteristic line "a" was obtained by plotting the change in value of the pumping electrode Ip2 when the NO concentration in a measurement gas ($O_2$ concentration = 0.3 %) was increased, based on the use of Example 1. A characteristic line "b" was obtained by plotting the change in value of the pumping electrode Ip2 when the NO concentration in a measurement gas ($O_2$ concentration = 20 %) was increased, based on the use of Example 1 as well. A characteristic line "c" was obtained by plotting the change in value of the pumping electrode Ip2 when the NO concentration in a measurement gas ($O_2$ concentration = 20 %) was increased, based on the use of Comparative Example 1.

According to the experimental results (FIG. 3), the detection sensitivity to NOx in Example 1 (Au-adding amount = 0.3 wt%) is not increased even when the oxygen concentration in the measurement gas in the external space is greatly changed from 0.3 % to 20 % as depicted by the characteristic line "b", as compared with the case of Comparative Example 1 (Au-adding amount = 0.1 wt%). Therefore, it is understood that the reaction of NO + $(1/2)O_2 \rightarrow NO_2$ is suppressed on the inner pumping electrode 64 and the auxiliary pumping electrode 90, as compared with Comparative Example 1.

Next, in the second illustrative experiment, the cermet electrode comprising the alloy of Au and the metal of the platinum group and the ceramic component such as $zrO_2$ was used to observe the NO decomposition ratio and the NO oxidation ratio obtained when the ratio of Au contained in the alloy was changed. Experimental results are shown in FIG. 4. In FIG. 4, a characteristic curve "a" depicts a characteristic of the change in NO decomposition ratio with respect to the change in Au-adding amount, and a characteristic curve "b" depicts a characteristic of the change in NO oxidation ratio with respect to the change in Au-adding amount. A division line (depicted as a chain line), which extends vertically, indicates an Au-adding amount = 0.5 wt%.

In the second illustrative experiment, the measurement was performed by using an NOx analyzer based on the measurement principle of the non-dispersion type infrared analysis method. In the NOx analyzer, the measurement condition for the NO decomposition ratio was NO concentration = 1000 ppm, $O_2$ concentration = 10 ppm, and gas temperature = 400°C. The measurement condition for the NO oxidation ratio was NO concentration = 1000 ppm, $O_2$ concentration = 20 %, and gas temperature = 400°C.

According to the experimental results shown in FIG. 4, the NO decomposition ratio was approximately zero in a range of the Au-adding amount of not less than 0.8 wt%, and both of the NO decomposition ratio and the NO oxidation ratio were approximately zero in a range of the Au-adding amount of not less than 8 wt%. According to this fact, it is understood that the reaction of NO + $(1/2)O_2 \rightarrow NO_2$ in the first chamber 60 can be diminished by optimizing the Au-adding amount.

Next, in the third illustrative experiment, observation was made for at what level the decomposition of NO occurred, the level being expressed by conversion into the electromotive force, when the correction for the reference voltage Vb effected by the feedback control system 70 was in an excessive correction state (indicated by the straight line "c" in FIG. 2), due to the increase in oxygen concentration. The decomposition of NO can be recognized from the fact that the value of the pumping electrode Ip2 flowing through the measuring pumping cell 82 is decreased to a level lower than a certain level.

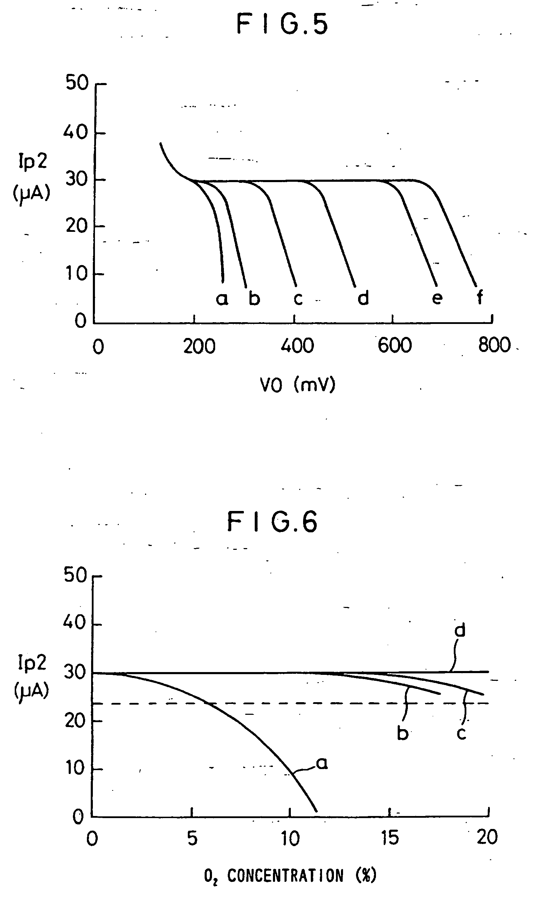

Experimental results are shown in FIG. 5. In FIG. 5, a characteristic curve "a" indicates a characteristic in the case of an Au-adding amount = 0, i.e., in the case of a Pt electrode. A characteristic curve "b" indicates a characteristic in the case of an Au-adding amount = 0.1 wt%. A characteristic curve "c" indicates a characteristic in the case of an Au-adding amount = 0.5 wt%. A characteristic curve "d" indicates a characteristic in the case of an Au-adding amount = 1 wt%. A characteristic curve "e" indicates a characteristic in the case of an Au-adding amount = 10 wt%. A characteristic curve "f" indicates a characteristic in the case of an Au-adding amount = 30 wt%. A region located rightward from an electromotive force = 300 mV indicates a control region concerning an atmosphere of a partial pressure of $O_2 = 1.3 \times 10^{-7}$ atm or more.

According to FIG. 5, it is understood that the decomposition of NO does not occur by appropriately adjusting the Au-adding amount even when the target value (= reference voltage Vb + control voltage Vd) of the pumping voltage Vp1 of the main pumping cell 68 is shifted toward the low oxygen concentration side.

Next, in the fourth illustrative experiment, observation was made for the change in sensitivity of the measuring pumping cell 82 (change in pumping electrode Ip2) with respect to the change in $O_2$ concentration (0 % to 20 %) in a measurement gas having an NO concentration of 5000 ppm, in the excessive correction state. Experimental results are shown in FIG. 6. In FIG. 6, a characteristic curve "a" indicates a characteristic in the case of an Au-adding amount = 0.1 wt%. A characteristic curve "b" indicates a characteristic in the case of an Au-adding amount = 0.5 wt%. A characteristic curve "c" indicates a characteristic in the case of an Au-adding amount = 1 wt%. A characteristic curve "d" indicates a characteristic in the case of an Au-adding amount = 10 to 30 wt%.

According to FIG. 6, it is understood that when the Au-adding amount is 0.1 wt%, the detection sensitivity to NOx is suddenly lowered when the oxygen concentration in the measurement gas becomes high, while when the Au-adding amount is not less than 0.5 wt%, the decrease in sensitivity is suppressed. When the Au-adding amount is 10 to 30 wt%, the detection sensitivity to NOx is scarcely changed even when the oxygen concentration is greatly changed, and the sensitivity is not affected by the change in oxygen concentration in the measurement gas.

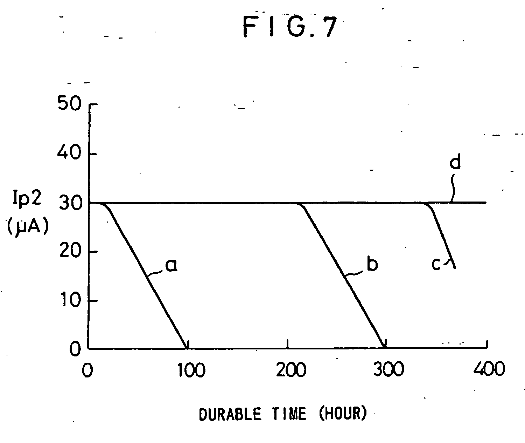

Next, in the fifth illustrative experiment, observation was made for the change in sensitivity of the measuring pumping cell 82 (change in pumping current Ip2) with respect to the operation time (= durable time: 0 to 400 hours) when the gas sensor 50A according to the first embodiment was operated in a measurement gas having an NO concentration of 5000 ppm. Experimental results are shown in FIG. 7. In FIG. 7, a characteristic curve "a" indicates a characteristic in the case of an Au-adding amount = 0.1 wt%. A characteristic curve "b" indicates a characteristic in the case of an Au-adding amount = 0.5 wt%. A characteristic curve "c" indicates a characteristic in the case of an Au-adding amount = 1 wt%. A characteristic curve "d" indicates a characteristic in the case of an Au-adding amount = 10 to 30 wt%.

According to FIG. 7, it is understood that when the Au-adding amount is 0.1 wt%, the detection sensitivity to NOx begins to decrease about 10 hours after the start of the operation, and the gas sensor 50A does not function after passage of time of 100 hours. However, when the Au-adding amount is 0.5 wt%, the detection sensitivity to NOx begins to decrease at a point of time after passage of a long period of time, i.e., about 200 hours after the start of the operation. In this case, the gas sensor 50A can be sufficiently put into practical use.

When the Au-adding amount is 1 wt%, the detection sensitivity to NOx begins to decrease about 330 hours

**EP 0 859 233 B1**

after the start of operation. When the Au-adding amount is 10 to 30 wt%, the decrease in detection sensitivity is not observed even after passage of 400 hours.

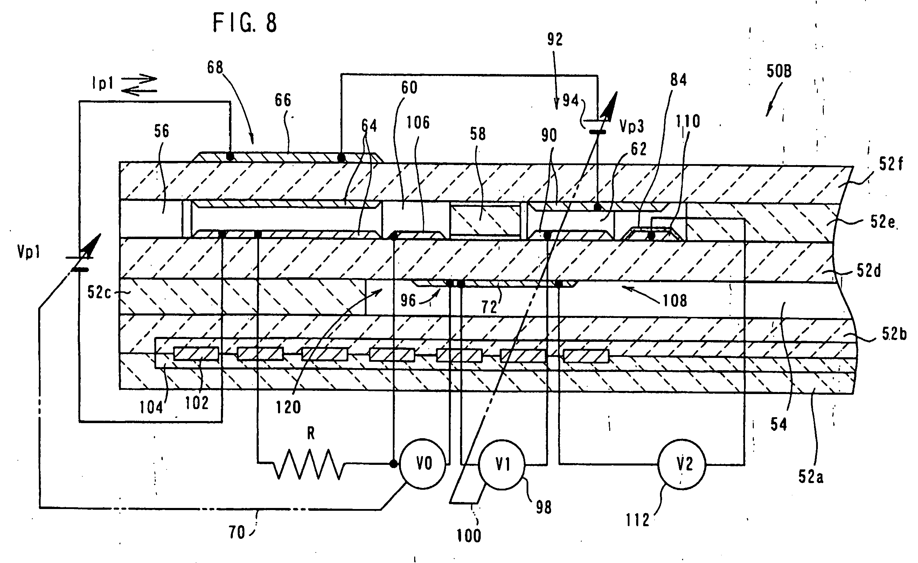

Next, a gas sensor 50B according to the second embodiment will be explained with reference to FIG. 8. Components or parts corresponding to those shown in FIG. 1 are designated by the same reference numerals, duplicate explanation of which will be omitted.

As shown in FIG. 8, the gas sensor 50B according to the second embodiment is constructed in approximately the same manner as the gas sensor 50A according to the first embodiment described above (see FIG. 1). However, the former is different from the latter in that a measuring electrode 106 is formed in the vicinity of the second diffusion rate-determining section 58 on an upper surface portion for forming the first chamber 60, of the upper surface of the first solid electrolyte layer 52d, a measuring oxygen partial pressure-detecting cell 108 is provided in place of the measuring pumping cell 82, and a resistor R is connected between the inner pumping electrode 64 and the measuring electrode 106. A controlling oxygen partial pressure-detecting cell 120 is constructed by the measuring electrode 106, the reference electrode 72, and the first solid electrolyte layer 52d.

The resistor R is provided for allowing the measurement gas to have an optimum partial pressure of oxygen by uniformalizing the oxygen concentration distribution in the depth direction in the first chamber 60 so that the measurement gas is supplied to the second chamber 62 without decomposing the objective component (NOx) in the measurement gas.

That is, if the oxygen concentration distribution is non-uniform, a current flows between the measuring electrode 106 and the inner pumping electrode 64, in an amount corresponding to the dispersion of concentration. The current is converted into a voltage by the aid of the resistor R, and the voltage is superimposed as a correction voltage on a target value (for example, 300 mV) for the feedback control system 70. The feedback control system 70 controls the pumping voltage Vp1 for the main pumping cell 68 so that the partial pressure of oxygen in the first chamber 60 is made constant, and the dispersion of the oxygen concentration distribution in the first chamber 60 is absorbed.

The measuring electrode 106 exposed to the inside of the first chamber 60 is a cermet electrode comprising an alloy of Au and a metal of the platinum group and a ceramic component, in the same manner as the inner pumping electrode 64 described above. Au is contained in the alloy in a ratio of not less than 0.3 wt% and not more than 35 wt%.

On the other hand, the measuring oxygen partial pressure-detecting cell 108 is constructed by a detecting electrode 110 formed on an upper surface portion for forming the second chamber 62, of the upper surface of the first solid electrolyte layer 52d, the reference electrode 72 formed on the lower surface of the first solid electrolyte layer 52d, and the first solid electrolyte layer 52d.

In this embodiment, an electromotive force (electromotive force of an oxygen concentration cell) V2, which corresponds to a difference in oxygen concentration between an atmosphere around the detecting electrode 110 and an atmosphere around the reference electrode 72, is generated between the detecting electrode 110 and the reference electrode 72 of the measuring oxygen partial pressure-detecting cell 108.

Therefore, the partial pressure of oxygen in the atmosphere around the detecting electrode 110, in other words, the partial pressure of oxygen defined by the oxygen produced by reduction or decomposition of the measurement gas component (NOx) is detected as a voltage value V2 by measuring the electromotive force (voltage) V2 generated between the detecting electrode 110 and the reference electrode 72 by using a voltmeter 112.

The degree of change in the electromotive force V2 represents the NO concentration. That is, the electromotive force V2, which is outputted from the measuring oxygen partial pressure-detecting cell 108 constructed by the detecting electrode 110, the reference electrode 72, and the first solid electrolyte layer 52d, represents the NO concentration in the measurement gas.

In the gas sensor 50B according to the second embodiment, each of the inner pumping electrode 64, the measuring electrode 106, and the auxiliary pumping electrode 90 is a cermet electrode comprising the alloy of Au and the metal of the platinum group and the ceramic component such as $ZrO_2$, and Au is contained in the alloy in a ratio of not less than 0.3 wt% and not more than 35 wt%, in the same manner as the gas sensor 50A according to the first embodiment described above.

Therefore, the inner pumping electrode 64, the measuring electrode 106, and the auxiliary pumping electrode 90, which include the alloy, have extremely low activities as the NOx-decomposing catalyst, and NOx is not decomposed even at a low partial pressure of oxygen. That is, the reaction of NO + $(1/2)O_2 \rightarrow NO_2$ is suppressed on the inner pumping electrode 64 in the first chamber 60, and the sensitivity to NO of the gas sensor 50B scarcely depends on the concentration of $O_2$ contained in the measurement gas.

Further, the Au-adding amount is defined to be not less than 0.3 wt% and not more than 35 wt%. Therefore, even when the Au component in the inner pumping electrode 64 of the main pumping cell 68 is decreased due to the operation of the gas sensor 50B for a long period of time, the catalytic activity of the inner pumping electrode 64 is not expressed.

As a result, in the gas sensor 50B according to the second embodiment described above, the oxygen, which behaves as a disturbing component for the measurement of NOx, can be excluded to be substantially zero without

13

exerting any influence on the measurement of NOx. Accordingly, it is possible to measure NOx contained in the measurement gas highly accurately and stably for a long period of time by the aid of the measuring pumping cell and the current-detecting means.

An illustrative experiment will now be described. In this illustrative experiment, there were prepared a gas sensor constructed in the same manner as the gas sensor 50B according to the second embodiment in which Au was added to the alloy for constructing the inner pumping electrode 64 in an amount of 0.3 wt% (Example 2), and a gas sensor constructed in the same manner as the gas sensor 50B according to the second embodiment in which Au was added in an amount of 0.1 wt% (Comparative Example 2), in the same manner as the first illustrative experiment mentioned above (see FIG. 3 for the experimental results). Observation was made for whether or not the reaction of NO + $(1/2)O_2 \rightarrow NO_2$ was suppressed by increasing the Au-adding amount.

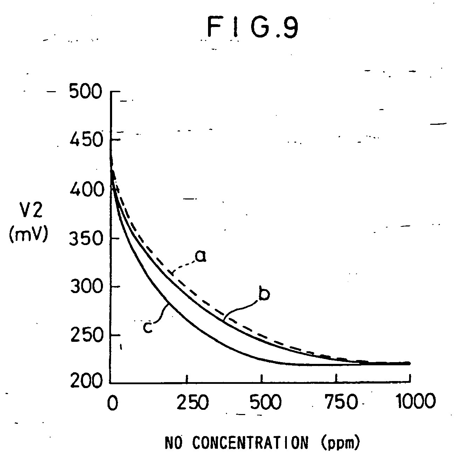

Experimental results are shown in FIG. 9. In FIG. 9, a characteristic curve "a" was obtained by plotting the change in the electromotive force V2 when the NO concentration in a measurement gas ($O_2$ concentration = 0.3 %) was increased, based on the use of Example 2. A characteristic curve "b" was obtained by plotting the change in the electromotive force V2 when the NO concentration in a measurement gas ($O_2$ concentration = 20 %) was increased, based on the use of Example 2 as well. A characteristic curve "c" was obtained by plotting the change in the electromotive force V2 when the NO concentration in a measurement gas ($O_2$ concentration = 20 %) was increased, based on the use of Comparative Example 2.

According to FIG. 9, the detection sensitivity to NOx in Example 2 (Au-adding amount = 0.3 wt%) is not increased even when the oxygen concentration in the measurement gas in the external space is greatly changed from 0.3 % to 20 % as depicted by the characteristic curve "b", as compared with the case of Comparative Example 2 (Au-adding amount = 0.1 wt%). Therefore, it is understood that the reaction of NO + $(1/2)O_2 \rightarrow NO_2$ is suppressed on the measuring electrode 106 and the auxiliary pumping electrode 90, as compared with Comparative Example 2.

Each of the gas sensors 50A, 50B according to the first and second embodiments are manufactured by stacking and integrating six green sheets composed of ceramics, followed by sintering.

Specifically, at first, a wiring pattern for the heater 102 is formed by printing via the insulative layer 104 on the first layer green sheet which serves as the first substrate layer 52a. Respective electrode pastes for the inner pumping electrode 64, the auxiliary pumping electrode 90, and the detecting electrode 80 (as well as the measuring electrode 106, if necessary) are formed by printing on a first principal surface of the fourth layer green sheet which serves as the first solid electrolyte layer 52d. An electrode paste for the reference electrode 72 is formed by printing on a second principal surface thereof.

After that, a ceramic layer, which serves as the third diffusion rate-determining section 84, is formed so as to cover the electrode paste for the detecting electrode 80 formed on the fourth layer green sheet.

Subsequently, electrode pastes for the inner pumping electrode 64 and the auxiliary pumping electrode 90 are formed by printing on side walls of respective windows (windows for comparting and forming the first chamber 60 and the second chamber 62) of the fifth layer green sheet which serves as the second spacer layer 52e.

After that, an electrode paste for the outer pumping electrode 66 is formed by printing on a first principal surface of the sixth layer green sheet which serves as the second solid electrolyte layer 52f. Respective electrode pastes for the inner pumping electrode 64 and the auxiliary pumping electrode 90 are formed by printing on a second principal surface thereof.

After that, the first to sixth green sheets are stacked one after another to construct a stacked unit, and the stacked unit is cut along a prescribed planar configuration. The stacked unit after the cutting is sintered and integrated at a temperature not less than 1300°C to produce the gas sensor 50A or 50B according to the first or second embodiment described above.

When the plurality of green sheets are stacked and integrated with each other by means of the sintering as described above to produce the gas sensor 50A or 50B, in which the cermet electrode comprising the alloy of Au and the metal of the platinum group and the ceramic component such as $ZrO_2$ is used for the inner pumping electrode 64 and the auxiliary pumping electrode 90, if the ratio of Au contained in the alloy is not less than 37 wt%, then the abnormal grain growth occurs in the alloy during the process of sintering and integration. For this reason, it is difficult to perform the sintering at a temperature not less than 1300°C. That is, under the foregoing condition, it is impossible to produce the gas sensor by sintering and integrating the stacked unit comprising the plurality of green sheets.

An illustrative experiment will now be described. In this illustrative experiment, observation was made for the change in electric resistance and the relationship between the Au-adding amount in the alloy for constructing the inner pumping electrode 64 and the presence or absence of the occurrence of abnormal grain growth. The presence or absence of the abnormal grain growth was determined by observing the electrode surface by means of SEM.

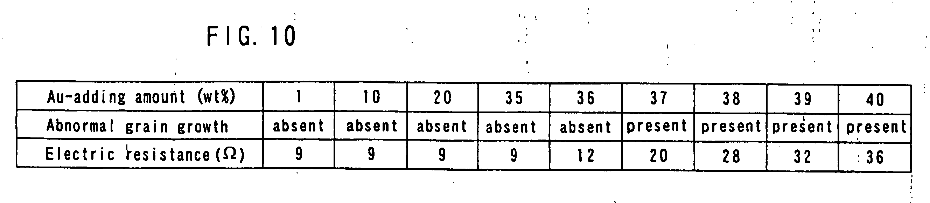

Experimental results are shown in a table in FIG. 10. According to the table in FIG. 10, it is understood that the abnormal grain growth occurs when the Au-adding amount is 37 wt% or more, in accordance with which the resistance value of the inner pumping electrode 64 is also increased. When the Au-adding amount was 36 wt%, the occurrence of abnormal grain growth was not observed. However, the electric resistance was 12 $\Omega$ which was higher than the resistance value of the electrode (9 Ω) obtained when the Au-adding amount was 35 wt% or less. Accordingly, it is assumed that when the Au-adding amount is 12 Ω, the abnormal grain growth occurs in a degree not observable by using SEM.

Therefore, the upper limit of the Au-adding amount is preferably 35 wt%, and optimally 10 wt%.

When the Au-adding amount is 0.2 wt%, the detection sensitivity to NO of the measuring pumping cell 82 is decreased in the excessive correction state as described above. Under this condition, when the gas sensor 50A is operated for a long period of time, the detection sensitivity to NO of the measuring pumping cell 82 is also decreased. Therefore, the lower limit of the Au-adding amount is not less than 0.3 wt%, and optimally 0.5 wt%.

In the gas sensors 50A, 50B according to the first and second embodiments described above, NOx is the objective as the measurement gas component. However, the present invention is also effectively applicable to measurement of bound oxygen-containing gas components other than NOx, for example, $H_2O$ and $CO_2$, which would be otherwise affected by the oxygen existing in the measurement gas.

**Claims**

1. A gas sensor for determining a predetermined gas component in a measurement gas by reducing or decomposing said predetermined gas component in said measurement gas to measure an amount of oxygen produced during said reduction or decomposition, said gas sensor having internal processing spaces (60, 62) and comprising:

    a main pumping means (68) including a pair of pumping electrodes (64, 66) one of which is disposed in a first said internal space (60) into which said measurement gas is introduced from external space, and the other of which is disposed in said external space, for pumping-processing oxygen contained in said measurement gas introduced from said external space, on the basis of a control voltage (Vp1) applied between said pair of pumping electrodes (64, 66) so that a partial pressure of oxygen in a process atmosphere is controlled to have a predetermined value at which NO is not decomposable;
    a measuring pumping means (82) including a pair of detecting electrodes (72, 80) one of which is disposed in a second said internal space (62) into which said measurement gas is introduced after being pumping-processed by said main pumping means (68), and the other of which is disposed in a reference gas introduction space (54), for pumping-processing oxygen contained in said measurement gas after being pumping-processed by said main pumping means (68), on the basis of a measuring voltage (Vp2) applied between said pair of detecting electrodes (72, 80); and
    a current-detecting means (88) for detecting a pumping current (Ip2) generated depending on an amount of said oxygen pumping-processed by said measuring pumping means (82); wherein
    at least one electrode (64) exposed to one of said internal spaces (60, 62) is a cermet electrode composed of an alloy of Au and a metal of the platinum group and a ceramic component,

    **characterised in that** Au is contained in said alloy in a ratio of not less than 0.3 wt% and not more than 35 wt%, and **in that** the gas sensor has an auxiliary pumping means (92) including a pair of auxiliary pumping electrodes (66, 90) one of which is formed in the vicinity of said detecting electrode (80 or 110) in said second internal space (62), and the other of which is disposed in said external space, for pumping-processing oxygen contained in said measurement gas after being pumping-processed by said main pumping means (68) on the basis of an auxiliary pumping voltage (Vp3) applied between said pair of auxiliary pumping electrodes (66, 90).

2. The gas sensor according to claim 1, wherein said oxygen is produced by either one of or both of actions of application of a voltage sufficient to decompose said predetermined gas component between said pair of detecting electrodes (72, 80), and a catalyst for decomposing said predetermined gas component, arranged for said measuring pumping means (82), and said measuring pumping means (82) pumping-processes said oxygen on the basis of said measuring voltage (Vp2) applied between said pair of detecting electrodes (72, 80).

3. A gas sensor for determining a predetermined gas component in a measurement gas by reducing or decomposing said predetermined gas component in said measurement gas to measure an amount of oxygen produced during said reduction or decomposition, said gas sensor having internal processing spaces (60, 62) and comprising:

    a main pumping means (68) including a pair of pumping electrodes (64, 66) one of which is disposed in a first said internal space (60) into which said measurement gas is introduced from external space, for pumping-processing oxygen contained in said measurement gas introduced from said external space, and the other of which is disposed in said external space, on the basis of a control voltage (Vp1) applied between said pair of pumping electrodes (64, 66) so that a partial pressure of oxygen in a process atmosphere is controlled to have a predetermined value at which NO is not decomposable;
a concentration-detecting means (108) including a pair of detecting electrodes (72, 110) one of which is disposed in a second said internal space into which said measurement gas is introduced after being pumping-processed by said main pumping means (68), and the other of which is disposed in a reference gas introduction space (54), for generating an electromotive force (V2) corresponding to a difference between an amount of oxygen contained in said measurement gas after being pumping-processed by said main pumping means (68) and an amount of oxygen contained in a gas existing in said reference gas introduction space (54); and
a voltage-detecting means (112) for detecting said electromotive force (V2) generated by said concentration-detecting means (108); wherein
at least one electrode (64) exposed to one of said internal spaces (60, 62) is a cermet electrode composed of an alloy of Au and a metal of the platinum group and a ceramic component,

**characterised in that** Au is contained in said alloy in a ratio of not les than 0.3 wt% and not more than 35 wt%, and **in that** the gas sensor has an auxiliary pumping means (92) including a pair of auxiliary pumping electrodes (66, 90) one of which is formed in the vicinity of said detecting electrode (80 or 110) in said second internal space (62), and the other of which is disposed in said external space, for pumping-processing oxygen contained in said measurement gas after being pumping-processed by said main pumping means (68) on the basis of an auxiliary pumping voltage (Vp3) applied between said pair of auxiliary pumping electrodes (66, 90).

4. The gas sensor according to claim 3, wherein said concentration detecting means (108) generates said electromotive force (V2) of an oxygen concentration cell corresponding to a difference between said oxygen produced in accordance with an action of a catalyst for decomposing said predetermined gas component arranged for said concentration-detecting means (108) and said oxygen contained in said gas existing in said reference gas introduction space (54).

5. The gas sensor according to any one of claims 1 to 4, wherein Au is contained in said alloy of Au and said metal of said platinum group in an amount of not less than 0.5 wt% and not more than 10 wt% with respect to said metal of said platinum group.

6. The gas sensor according to any one of claims 1 to 5, wherein a concentration-measuring means (120) including a pair of measuring electrodes (64, 72; 72, 106) one of which is disposed in said first internal space (60), and the other of which his disposed in a reference gas introduction space (54), for measuring an electromotive force (V0) generated depending on a difference between an amount of oxygen contained in said measurement gas during said pumping process effected by said main pumping means (68) and an amount of oxygen contained in an atmosphere at the other measuring electrode (72); and
a main pumping control means (70) for adjusting said control voltage (Vp1) for said main pumping means (68) on the basis of said electromotive force (V0) detected by said concentration-measuring means.

7. The gas sensor according to claim 6, wherein said main pumping control means (70) is provided with a comparing means (74) for determining a deviation (Vc) between said electromotive force (V0) and a comparative voltage (Vb), and with means for adjusting the level of said control voltage (Vp1) applied between said pair of pumping electrodes (64, 66) of said main pumping means (68) on the basis of said deviation (Vc) obtained by said comparing means (74).

8. The gas sensor according to claim 7, further comprising:

   a pumping current-detecting means (Ri) for detecting a pumping current (Ip1) flowing through said main pumping means (68) during said pumping process effected by said main pumping means (68); and
   a comparative voltage-correcting means (78) for correcting a level of said comparative voltage (Vb) on the basis of a value of said pumping current (Ip1) detected by said pumping current-detecting means (Ri).

9. The gas sensor according to any one of claims 6 to 8, wherein said one pumping electrode (64) of said main pumping means (68) is commonly used as said one measuring electrode (106) of said concentration-measuring means (120).

10. The gas sensor according to any one of claims 1 to 9, wherein the other auxiliary pumping electrode (66) is commonly used as said outer pumping electrode (66) of said main pumping means (68).

11. The gas sensor according to any one of claims 1 to 10, further comprising:

    an auxiliary concentration-measuring means (96) including a pair of auxiliary measuring electrodes (72, 90) one of which is disposed in said second internal space (62), and the other of which is disposed in a reference gas introduction space (54), for detecting an electromotive force (V1) generated corresponding to a difference between an amount of oxygen contained in said measurement gas after being pumping-processed by said main pumping means (68) and an amount of oxygen contained in a gas existing in said reference gas introduction space (54); and

an auxiliary pumping control means (100) for adjusting said auxiliary pumping voltage (Vp3) for said auxiliary pumping means (92) on the basis of said electromotive force (V1) detected by said auxiliary concentration-measuring means (96).

**12.** The gas sensor according to claim 11, wherein:

said one auxiliary measuring electrode (90) is commonly used as said auxiliary pumping electrode (90) of said auxiliary pumping means (92); and

the other auxiliary measuring electrode (72) is commonly used as the other detecting electrode (72) of said measuring pumping means (82) or said concentration-detecting means (108).

**13.** The gas sensor according to any one of claims 1 to 12, wherein:

said plurality of electrodes (64, 66, 72, 80, 90, 106, 110) for constructing said respective means are formed on a substrate including solid electrolytes (52a to 52f); and

said substrate is constructed by forming an insulative layer and an electrode layer on a green sheet composed of said solid electrolyte, stacking and integrating a plurality of said green sheets into one stacked unit, and sintering said stacked unit.

**Patentansprüche**

**1.** Gassensor zum Bestimmen einer vorbestimmten Gaskomponente in einem Messgas durch Reduzieren oder Zersetzen der vorbestimmten Gaskomponente in dem Messgas, um eine während der Reduktion oder der Zersetzung erzeugte Sauerstoffmenge zu messen, wobei der Gassensor Innenverarbeitungsräume (60, 62) aufweist und Folgendes umfasst:

ein Hauptpumpmittel (68), das ein Paar Pumpelektroden (64,66) umfasst, von denen die eine in einem ersten Innenraum (60) angeordnet ist, in den das Messgas aus einem Außenraum eingeleitet wird, und von denen die andere in dem Außenraum angeordnet ist, zur Pumpverarbeitung von in dem aus dem Außenraum eingeleiteten Messgas enthaltenen Sauerstoff auf Basis einer Steuerspannung (Vp1), die zwischen dem Paar Pumpelektroden (64, 66) angelegt wird, so dass ein Sauerstoffpartialdruck in einer Verarbeitungsumgebung gesteuert wird, um einen vorbestimmten Wert zu haben, bei dem NO nicht zersetzbar ist;

ein Messpumpmittel (82), das ein Paar Detektionselektroden (72, 80) umfasst, von denen die eine in einem zweiten Innenraum (62) angeordnet ist, in der das Messgas nach der Pumpverarbeitung durch das Hauptpumpmittel (68) eingeleitet wird, und von denen die andere in einem Referenzgas-Einleitungsraum (54) angeordnet ist, zur Pumpverarbeitung des in dem Messgas enthaltenen Sauerstoffs nach der Pumpverarbeitung durch das Hauptpumpmittel (68) auf Basis einer Messspannung (Vp2), die zwischen dem Paar Detektionselektroden (72, 80) angelegt wird; und

ein StromdetektionsmitteJ (88) zum Detektieren eines Pumpstroms (Ip2), der in Abhängigkeit von einer Menge des durch das Messpumpmittel (82) pumpverarbeiteten Sauerstoffs erzeugt wird; wobei

zumindest eine Elektrode (64), die gegenüber einem der Innenräume (60, 62) freiliegt, eine Cermetelektrode ist, die aus einer Legierung aus Au und einem Metall der Platingruppe und einer Keramikkomponente zusammengesetzt ist,

**dadurch gekennzeichnet, dass** Au in der Legierung in einem Verhältnis von nicht weniger als 0,3 Gew.-% und nicht mehr als 35 Gew.-% erhalten ist und

dass der Gassensor ein Hilfspumpmittel (92) aufweist, das ein Paar Hilfspumpelektroden (66, 90) umfasst, von denen die eine in der Nähe der Detektionselektrode (80 oder 110) im zweiten Innenraum (62) ausgebildet ist und von denen die andere in dem Außenraum (62) angeordnet ist, zur Pumpverarbeitung des in dem Messgas enthaltenen Sauerstoffs nach der Pumpverarbeitung durch das Hauptpumpmittel (68) auf Basis einer Hilfspumpspannung (Vp3), welche zwischen dem Paar an Hilfspumpelektroden (66, 90) angelegt wird.

2. Gassensor nach Anspruch 1, worin der Sauerstoff entweder durch einen oder beide Vorgänge des Anlegens einer Spannung erzeugt wird, die ausreicht, um die vorbestimmte Gaskomponente zwischen dem Paar Detektionselektroden (72, 80) zu zersetzen, wobei ein Katalysator zum Zersetzen der vorbestimmten Gaskomponente für das Messpumpmittel (82) angeordnet ist, und wobei das Messpumpmittel (82) den Sauerstoff auf Basis der Messspannung (Vp2), welche zwischen dem Paar Detektionselektroden (72, 80) angelegt wird, pump-verarbeitet.

3. Gasssensor zum Bestimmen einer vorbestimmten Gaskomponente in einem Messgas durch Reduzieren oder Zersetzen der vorbestimmten Gaskomponente in dem Messgas, um eine während der Reduktion oder der Zersetzung erzeugte Sauerstoffmenge zu messen, wobei der Gassensor Innenverarbeitungsräume (60, 62) aufweist und Folgendes umfasst:

ein Hauptpumpmittel (68), das ein Paar Pumpelektroden (64, 66) umfasst, von denen die eine in einem ersten Innenraum (60), in den das Messgas aus einem Außenraum eingeleitet wird, angeordnet ist, zur Pumpverarbeitung des in dem Messgas enthaltenen Sauerstoffs, der aus dem Außenraum eingeleitet wird, und von denen die andere in dem Außenraum angeordnet ist, auf Basis einer Steuerspannung (Vp1), die zwischen dem Paar Pumpelektroden (64, 66) angelegt wird, so dass ein Sauerstoffpartialdruck in einer Verarbeitungsumgebung gesteuert wird, um einen vorbestimmten Wert zu haben, bei dem NO nicht zersetzbar ist;
ein Konzentrationsdetektionsmittel (108), das ein Paar Detektionselektroden (72, 110) umfasst, von denen die eine in dem zweiten Innenraum angeordnet ist, in den das Messgas nach der Pumpverarbeitung durch das Hauptpumpmittel (68) eingeleitet wird, und von denen die andere in einem Referenzgas-Einleitungsraum (54) angeordnet ist, zur Erzeugung einer elektromotorischen Kraft (V2), welche einer Differenz zwischen einer in dem Messgas nach der Pumpverarbeitung durch das Hauptpumpmittel (68) enthaltenen Sauerstoffmenge und einer in einem Gas, das in dem Referenzgas-Einleitungsraum (54) vorliegt, enthaltenen Sauerstoffmenge entspricht; und
ein Spannungsdetektionsmittel (112) zur Detektion der elektromotorischen Kraft (V2), welche durch das Konzentrationsdetektionsmittel (108) erzeugt wird; wobei
zumindest eine Elektrode (64), die gegenüber einem der Innenräume (60, 62) freiliegt, eine Cermetelektrode ist, die aus einer Legierung aus Au und einem Metall der Platingruppe und einer Keramikkomponente zusammengesetzt ist,

dadurch gekennzeichnet, dass Au in der Legierung in einem Verhältnis von nicht weniger als 0,3 Gew.-% und nicht mehr als 35 Gew.-% enthalten ist und
dass der Gassensor ein Hilfspumpmittel (92) aufweist, das ein Paar Hilfspumpelektroden (66, 90) umfasst, von denen die eine in der Nähe der Detektiondelektrode (80 oder 110) im zweiten Innenraum (62) ausgebildet ist und von denen die andere in dem Außenraum angeordnet ist, zur Pumpverarbeitung des in dem Messgas enthaltenen Sauerstoffs nach der Pumpverarbeitung durch das Hauptpumpmittel (68) auf Basis einer Hilfspumpspannung (Vp3), welche zwischen dem Paar an Hilfspumpelektroden (66, 90) angelegt wird.

4. Gassensor nach Anspruch 3, worin das Konzentrationsdetektionsmittel (108) die elektromotorische Kraft (v2) einer Sauerstoffkonzentrationszelle erzeugt, die einer Differenz zwischen dem in Übereinstimmung mit einer Wirkung eines Katalysators zur Zersetzung der vorbestimmten Gaskomponente, die für das Konzentrationsdetektionsmittel (108) angeordnet ist, erzeugten Sauerstoff und dem in dem Gas, welches in dem Referenzgas-Einleitungsraum (54) vorliegt, enthaltenen Sauerstoff entspricht.

5. Gassensor nach einem der Ansprüche 1 bis 4, worin Au in der Legierung von Au und dem Metall aus der Platingruppe in einer Menge von nicht weniger als 0,5 Gew.-% und nicht mehr als 10 Gew.-% in Bezug auf das Metall aus der Platingruppe enthalten ist.

6. Gassensor nach einem der Ansprüche 1 bis 5, worin ein Konzentrationsmessmittel (120) ein Paar Messelektroden (64, 72; 72, 106) umfasst, von denen die eine in dem ersten Innenraum (60) angeordnet ist und von denen die andere in einem Referenzgas-Einleitungsraum (54) angeordnet ist, zum Messen einer elektromotorischen Kraft (V0), welche in Abhängigkeit von einer Differenz zwischen einer in dem Messgas während des Pumpvorgangs, der durch das Hauptpumpmittel (68) ausgeführt wird, enthaltenen Sauerstoffs und einer in einer Umgebung an der anderen Messelektrode (72) enthaltenen Sauerstoffs erzeugt wird; und
ein Hauptpump-Steuermittel (70) zum Einstellen der Steuerspannung (Vp1) für das Hauptpumpmittel (68) auf Basis der elektromotorischen Kraft (V0), die durch das Konzentrationsmessmittel detektiert wird.

7. Gassensor nach Anspruch 6, worin das Hauptpump-Steuermittel (70) mit einem Vergleichsmittel (74) zum Bestimmen einer Abweichung (Vc) zwischen der elektromotorischen Kraft (V0) und einer Vergleichsspannung (Vb) bereitgestellt ist, und mit Mitteln zum Einstellen des Pegels der Steuerspannung (Vp1) bereitgestellt ist, die zwischen dem Paar Pumpelektroden (64, 66) des Hauptpumpmittels (68) auf Basis der Abweichung (Vc) angelegt wird, die durch das Vergleichsmittel (74) erhalten wird.

8. Gassensor nach Anspruch 7, ferner umfassend:

ein Pumpstrom-Detektionsmittel (Ri) zum Detektieren eines Pumpstroms (Ip1), der während des durch das Hauptpumpmittel (68) bewirkten Pumpvorgangs durch das Hauptpumpmittel (68) fließt; und
ein Vergleichsspannungs-Korrekturmittel (78) zum Korrigieren eines Pegels der Vergleichsspannung (Vb) auf Basis eines Werts des Pumpstroms (Ip1), der durch das Pumpstrom-Detektionsmittel (Ri) detektiert wird.

9. Gassensor nach einem der Ansprüche 6 bis 8, worin die eine Pumpelektrode (64) des Hauptpumpmittels (68) im Allgemeinen als die eine Messelektrode (106) des Konzentrationsmessmittels (120) verwendet wird.

10. Gassensor nach einem der Ansprüche 1 bis 9, worin die andere Hilfspumpelektrode (66) im Allgemeinen als die Außenpumpelektrode (66) des Hauptpumpmittels (68) verwendet wird.

11. Gassensor nach einem der Ansprüche 1 bis 10, ferner umfassend:

ein Hilfskonzentrations-Messmittel (96), das ein Paar Hilfsmesselektroden (72, 90) umfasst, von denen die eine in dem zweiten Innenraum (62) angeordnet ist und von denen die andere in einem Referenzgas-Einleitungsraum (54) zur Detektion einer elektromotorischen Kraft (V1) angeordnet ist, die entsprechend einer Differenz zwischen einer in dem Messgas nach der Pumpverarbeitung durch das Hauptpumpmittel (68) enthaltenen Sauerstoffmenge und einer in einem Gas, das in dem Referenzgas-Einleitungsraum (54) vorliegt, enthaltenen Sauerstoffmenge erzeugt wird; und
ein Hilfspump-Steuermittel (100) zum Einstellen der Hilfspumpspannung (Vp3) für das Hilfspumpmittel (92) auf Basis der elektromotorischen Kraft (V1), die durch das Hilfskonzentrations-Messmittel (96) detektiert wird.

12. Gassensor nach Anspruch 11, worin:

die eine Hilfsmesselektrode (90) im Allgemeinen als die Hilfspumpelektrode (90) des Hilfspumpmittels (92) verwendet wird; und
die andere Hilfsmesselektrode (72) im Allgemeinen als die andere Detektionselektrode (72) des Messpumpmittels (82) oder des Konzentrationsdetektionsmittels (108) verwendet wird.

13. Gassensor nach einem der Ansprüche 1 bis 12, worin:

die Vielzahl an Elektroden (64, 66, 72, 80, 90, 106, 110) zum Bilden der jeweiligen Mitteln auf einem Substrat, das Festkörperelektrolyte (52a bis 52f) umfasst, ausgebildet ist; und
das Substrat durch Ausbilden einer Isolierschicht und einer Elektrodenschicht auf einer grünen Lage, bestehend aus dem Festkörperelektrolyt, durch Stapeln und einstückiges Ausbilden einer Vielzahl an grünen Lagen in einer einzigen gestapelten Einheit und durch Sintern der gestapelten Einheit gebildet wird.

## Revendications

1. Capteur de gaz pour déterminer un composant de gaz prédéterminé dans un gaz de mesure en réduisant ou en décomposant ledit composant de gaz prédéterminé dans ledit gaz de mesure pour mesurer une quantité d'oxygène produit pendant ladite réduction ou décomposition, ledit capteur de gaz ayant des espaces de traitement internes (60, 62) et comprenant :

un moyen de pompage principal (68) comprenant une paire d'électrodes de pompage (64, 66) dont l'une est disposée dans un premier dit espace interne (60) dans lequel ledit gaz de mesure est introduit à partir d'un espace externe, et dont l'autre est disposée dans ledit espace externe, pour traiter par pompage l'oxygène contenu dans ledit gaz de mesure introduit à partir dudit espace externe, sur la base d'une tension de commande (Vp1) appliquée entre ladite paire d'électrodes de pompage (64, 66) de sorte qu'une pression partielle d'oxygène dans une atmosphère de traitement est régulée pour avoir une valeur prédéterminée à laquelle le NO n'est pas décomposable;

un moyen de pompage de mesure (82) comprenant une paire d'électrodes de détection (72, 80) dont l'une est disposée dans un second dit espace interne (62) dans lequel ledit gaz de mesure est introduit après avoir été traité par pompage par ledit moyen de pompage principal (68), et dont l'autre est disposée dans un espace d'introduction de gaz de référence (54), pour traiter par pompage l'oxygène contenu dans ledit gaz de mesure après avoir été traité par pompage par ledit moyen de pompage principal (68), sur la base d'une tension de mesure (Vp2) appliquée entre ladite paire d'électrodes de détection (72, 80); et

un moyen de détection de courant (88) pour détecter un courant de pompage (Ip2) généré selon une quantité dudit oxygène traitée par pompage par ledit moyen de pompage de mesure (82);

dans lequel

au moins une électrode (64) exposée à l'un desdits espaces internes (60, 62) est une électrode en cermet composée d'un alliage d'Au et d'un métal du groupe platine et d'un composant céramique,

**caractérisé en ce que** l'Au est contenu dans ledit alliage en un.rapport non inférieur à 0,3 % en poids et non supérieur à 35 % en poids, et

**en ce que** le capteur de gaz comporte un moyen de pompage auxiliaire (92) comprenant une paire d'électrodes de pompage auxiliaire (66, 90) dont l'une est formée au voisinage de ladite électrode de détection (80 ou 110) dans ledit second espace interne (62), et dont l'autre est disposée dans ledit espace externe, pour traiter par pompage l'oxygène contenu dans ledit gaz de mesure après avoir été traité par pompage par ledit moyen de pompage principal (68) sur la base d'une tension de pompage auxiliaire (Vp3) appliquée entre ladite paire d'électrodes de pompage auxiliaire (66, 90).

2. Capteur de gaz selon la revendication 1, dans lequel ledit oxygène est produit par l'une ou l'autre ou les deux actions d'application d'une tension suffisante pour décomposer ledit composant de gaz prédéterminé entre ladite paire d'électrodes de détection (72, 80), et un catalyseur pour décomposer ledit composant de gaz prédéterminé, agencé pour ledit moyen de pompage de mesure (82), et ledit moyen de pompage de mesure (82) traite par pompage ledit oxygène sur la base de ladite tension de mesure (Vp2) appliquée entre ladite paire d'électrodes de détection (72, 80).

3. Capteur de gaz pour déterminer un composant de gaz prédéterminé dans un gaz de mesure en réduisant ou en décomposant ledit composant de gaz prédéterminé dans ledit gaz de mesure pour mesurer une quantité d'oxygène produit pendant ladite réduction ou décomposition, ledit capteur de gaz ayant des espaces de traitement internes (60, 62) et comprenant:

un moyen de pompage principal (68) comprenant une paire d'électrodes de pompage (64, 66) dont l'une est disposée dans un premier dit espace interne (60) dans lequel ledit gaz de mesure est introduit à partir d'un espace externe, pour traiter par pompage l'oxygène contenu dans ledit gaz de mesure introduit à partir dudit espace externe, et dont l'autre est disposé dans ledit espace externe, sur la base d'une tension de commande (Vp1) appliquée entre ladite paire d'électrodes de pompage (64, 66) de sorte qu'une pression partielle d'oxygène dans une atmosphère de traitement est régulée pour avoir une valeur prédéterminée à laquelle le NO n'est pas décomposable;

un moyen de détection de concentration (108) comprenant une paire d'électrodes de détection (72, 110) dont l'une est disposée dans un second dit espace interne dans lequel ledit gaz de mesure est introduit après avoir été traité par pompage par ledit moyen de pompage principal (68), et dont l'autre est disposée dans un espace d'introduction de gaz de référence (54), pour générer une force électromotrice (V2) correspondant à une différence entre une quantité d'oxygène contenu dans ledit gaz de mesure après avoir été traité par pompage par ledit moyen de pompage principal (68) et une quantité d'oxygène contenu dans un gaz existant dans ledit espace d'introduction de gaz de référence (54); et

un moyen de détection de tension (112) pour détecter ladite force électromotrice (V2) générée par ledit moyen de détection de concentration (108); dans lequel

au moins une électrode (64) exposée à l'un desdits espaces internes (60, 62) est une électrode en cermet composée d'un alliage d'Au et d'un métal du groupe platine et d'un composant céramique,

**caractérisé en ce que** l'Au est contenu dans ledit alliage en un rapport non inférieur à 0,3 % en poids et non supérieur à 35 % en poids, et

**en ce que** le capteur de gaz comporte un moyen de pompage auxiliaire (92) comprenant une paire d'électrodes de pompage auxiliaire (66, 90) dont l'une est formée au voisinage de ladite électrode de détection (80 ou 110) dans ledit second espace interne (62), et dont l'autre est disposée dans ledit espace externe, pour traiter par pompage l'oxygène contenu dans ledit gaz de mesure après avoir été traité par pompage par ledit moyen de pompage principal

EP 0 859 233 B1

(68) sur la base d'une tension de pompage auxiliaire (Vp3) appliquée entre ladite paire d'électrodes de pompage auxiliaire (66, 90).

4. Capteur de gaz selon la revendication 3, dans lequel ledit moyen de détection de concentration (108) génère ladite force électromotrice (V2) d'une cellule de concentration en oxygène correspondant à une différence entre ledit oxygène produit selon une action d'un catalyseur pour décomposer ledit composant de gaz prédéterminé agencé pour ledit moyen de détection de concentration (108) et ledit oxygène contenu dans ledit gaz existant dans ledit espace d'introduction de gaz de référence (54).

5. Capteur de gaz selon l'une quelconque des revendications 1 à 4, dans lequel l'Au est contenu dans ledit alliage d'Au et ledit métal dudit groupe platine en une quantité non inférieure à 0,5 % en poids et non supérieure à 10 % en poids par rapport audit métal dudit groupe platine.

6. Capteur de gaz selon l'une quelconque des revendications 1 à 5, dans lequel un moyen de mesure de concentration (120) comprend une paire d'électrodes de mesure (64, 72; 72, 106) dont l'une est disposée dans ledit premier espace interne (60), et dont l'autre est disposée dans un espace d'introduction de gaz de référence (54), pour mesurer une force électromotrice (V0) générée selon une différence entre une quantité d'oxygène contenu dans ledit gaz de mesure pendant ledit traitement par pompage effectué par ledit moyen de pompage principal (68) et une quantité d'oxygène contenu dans une atmosphère au niveau de l'autre électrode de mesure (72); et un moyen de commande de pompage principal (70) pour ajuster ladite tension de commande (Vp1) pour ledit moyen de pompage principal (68) sur la base de ladite force électromotrice (V0) détectée par ledit moyen de mesure de concentration.

7. Capteur de gaz selon la revendication 6, dans lequel ledit moyen de commande de pompage principal (70) est doté d'un moyen de comparaison (74) pour déterminer un écart (Vc) entre ladite force électromotrice (V0) et une tension comparative (Vb), et d'un moyen pour ajuster le niveau de ladite tension de commande (Vp1) appliquée entre ladite paire d'électrodes de pompage (64, 66) dudit moyen de pompage principal (68) sur la base dudit écart (Vc) obtenu par ledit moyen de comparaison (74).

8. Capteur de gaz selon la revendication 7, comprenant en outre:

un moyen de détection de courant de pompage (Ri) pour détecter un courant de pompage (Ip1) circulant à travers ledit moyen de pompage principal (68) pendant ledit traitement par pompage effectué par ledit moyen de pompage principal (68); et
un moyen de correction de tension comparative (78) pour corriger un niveau de ladite tension comparative (Vb) sur la base d'une valeur dudit courant de pompage (Ip1) détecté par ledit moyen de détection de courant de pompage (Ri).

9. Capteur de gaz selon l'une quelconque des revendications 6 à 8, dans lequel ladite une électrode de pompage (64) dudit moyen de pompage principal (68) est communément utilisée comme ladite une électrode de mesure (106) dudit moyen de mesure de concentration (120).

10. Capteur de gaz selon l'une quelconque des revendications 1 à 9, dans lequel l'autre électrode de pompage auxiliaire (66) est communément utilisée comme ladite électrode de pompage externe (66) dudit moyen de pompage principal (68).

11. Capteur de gaz selon l'une quelconque des revendications 1 à 10, comprenant en outre:

un moyen de mesure de concentration auxiliaire (96) comprenant une paire d'électrodes de mesure auxiliaire (72, 90) dont l'une est disposée dans ledit second espace interne (62), et dont l'autre est disposée dans un espace d'introduction de gaz de référence (54), pour détecter une force électromotrice (V1) générée correspondant à une différence entre une quantité d'oxygène contenu dans ledit gaz de mesure après avoir été traité par pompage par ledit moyen de pompage principal (68) et une quantité d'oxygène contenu dans un gaz existant dans ledit espace d'introduction de gaz de référence (54); et
un moyen de commande de pompage auxiliaire (100) pour ajuster ladite tension de pompage auxiliaire (Vp3) pour ledit moyen de pompage auxiliaire (92) sur la base de ladite force électromotrice (V1) détectée par ledit moyen de mesure de concentration auxiliaire (96).

**12.** Capteur de gaz selon la revendication 11, dans lequel:

ladite électrode de mesure auxiliaire (90) est communément utilisée comme ladite électrode de pompage auxiliaire (90) dudit moyen de pompage auxiliaire (92); et
l'autre électrode de mesure auxiliaire (72) est communément utilisée comme l'autre électrode de détection (72) dudit moyen de pompage de mesure (82) ou dudit moyen de détection de concentration (108).

**13.** Capteur de gaz selon l'une quelconque des revendications 1 à 12, dans lequel:

ladite pluralité d'électrodes (64, 66, 72, 80, 90, 106, 110) pour construire ledit moyen respectif est formée sur un substrat comprenant les électrolytes solides (52a à 52f); et
ledit substrat est construit en formant une couche isolante et une couche d'électrode sur une feuille crue composée dudit électrolyte solide, en empilant et en intégrant une pluralité desdites feuilles crues en une unité empilée, et en frittant ladite unité empilée.

FIG. 1

EP 0 859 233 B1

# FIG.2

INCREASE IN OXYGEN CONCENTRATION

# F I G.3

NO CONCENTRATION (ppm)

# F I G.4

Au-ADDING AMOUNT (wt%)

25

# F I G.5

# F I G.6

# FIG.7

DURABLE TIME (HOUR)

FIG. 8

# F I G .9

FIG. 10

| Au-adding amount (wt%) | 1 | 10 | 20 | 35 | 36 | 37 | 38 | 39 | 40 |
|---|---|---|---|---|---|---|---|---|---|
| Abnormal grain growth | absent | absent | absent | absent | absent | present | present | present | present |
| Electric resistance (Ω) | 9 | 9 | 9 | 9 | 12 | 20 | 28 | 32 | 36 |

# FIG.11

EP 0 859 233 B1

**EP 0 859 233 B1**

**Patent documents cited in the description**

- WO 9530146 A
- EP 678740 A
- EP 0731351 A